Europäisches Patentamt

European Patent Office

Office européen des brevets

(11) Publication number: **0 248 147**
A2

# EUROPEAN PATENT APPLICATION

(21) Application number: 87102960.9

(22) Date of filing: 03.03.87

(51) Int. Cl.³: **G 01 N 33/574**
G 01 N 33/80, G 01 N 33/566
G 01 N 33/577, G 01 N 33/52

(30) Priority: 04.03.86 US 835953

(43) Date of publication of application:
09.12.87 Bulletin 87/50

(84) Designated Contracting States:
DE GB

(71) Applicant: Sloan-Kettering Institute For Cancer Researc
1275 York Avenue
New York New York 10021(US)

(72) Inventor: Sakamoto, Junichi
404 Minamigaoka Iris 1-10-62 Minamigaoka
Chikusaku Nagoya(JP)

(72) Inventor: Furukawa, Koichi
504 East 81 Street
New York New York 10028(US)

(72) Inventor: Cordon-Cardo, Carlos
430 East 67 Street
New York New York 10021(US)

(72) Inventor: Yin, Beatrice W.T.
136-76 72 Avenue
Flushing New York 11367(US)

(72) Inventor: Rettig, Wolfgang J.
347 East 61 Street
New York New York 10021(US)

(72) Inventor: Oettgen, Herbert F.
58 Overlook Drive
New Canaan Connecticut 06840(US)

(72) Inventor: Old, Lloyd J.
600 West End Avenue
New York New York 10024(US)

(72) Inventor: Lloyd, Kenneth O.
4525 Henry Hudsn parkway West
Bronx New York 10471(US)

(74) Representative: Henkel, Feiler, Hänzel & Partner
Möhlstrasse 37
D-8000 München 80(DE)

(54) Method of determining the presence of cancer cells.

(57) Panels of monoclonal antibodies specific for blood group antigens are used to detect the presence of cancer in samples. It is observed that expression of particular blood group antigens is associated with specific cancer conditions.

SK 387-JEL/NDH

0248147

## FIELD OF THE INVENTION

This invention relates to monoclonal antibodies which are useful in the diagnosis of cancer, the hybridomca cell lines which produce them, and methods employing the use of these monoclonal antibodies.

## BACKGROUND AND PRIOR ART

Blood group antigens, the source of the term "blood type" are molecules formed by sequential addition of saccharides to carbohydrate side chains of lipids and proteins. Two types of backbone structure are known: "type 1" chains, which have the structure Gal $\beta$ (1 $\longrightarrow$ 3)GlcNAc, and "type 2" chains, with structure Gal$\beta$(1 $\longrightarrow$ 4)GlcNAc. Kabat, in Carbohydrates in Solution (H.S. Isabel, ed.), Adv. Chem., 117, pp. 334-361 (Am. Chem. Soc. 1973) (Washington, D.C.); Watkins, in Advances in Human Genetics (H. Harris & K. Hirschorn, ed.) vol. 10, pp. 1-136 (1980); Lloyd, in International Review of Science, Organic Chemistry Series Two (G.O. Aspinall, ed.), vol. 7, pp. 252-281 (Butterworth, London) (1976). Glycosylation of the first structure leads to the Lewis a, Lewis b and H-1 antigens, while fucosylation of type 2 results in X, Y and H-2 determinants. When $\alpha$-D-GalNAc, or $\alpha$-D-Gal is added to Lewis b, Y, or H antigens, the A and B type antigens, respectively, are produced. Other variants on these primary structures have been demonstrated to be present on erythrocytes. These variants are designated as Type 3, and Type 4, respectively

SK 387-JEL/NDH

0248147

Clausen, et al., PNAS 82:1199-1203; Clausen, et al. Brochem Biophys. Res. Commin. 124:523-529 (1984).

Synthesis of A, B, and H antigens is accomplished by erythrocytes, followed by intergration on cell membranes. Cartron, et al. Blood Trans. Immunohematol 23:271-284 (1980). Lewis antigens are not synthesized by erythrocytes, and their site of synthesis is not known. They are, however, absorbed from plasma onto red blood cell surfaces, and reside on endothelial cells, and on some epithelial cells. They are shed, in the form of bound or free glycoprotein oligosaccharides, into body fluids such as pancreatic secretions and saliva. Sakamoto, et al., Molec Immunnol 21: 1093-1098 (1984); Watkins, Science 152:172-175 (1966).

While these antigens have been termed "blood group antigens," the term is misleading. Distribution of these antigens is not confined to red cells. Hakomori, et al., in The Antigens, vol. 2, pp. 79-140 (M. Sela ed., New York, Academic Press) (1974), have observed their presence in epithelial secretions. See also Marcus New Eng. J. Med. 280: 994-1006 (1969). Tumors, especially carcinomas, may produce blood group antigens, and it has been observed that the expression of these antigens is often modified, either in the direction of simplification of structure, or expression of anomalous specifities. Hakomori, et. al., J. Natl. Cancer Inst. 71:231-251 (1982). Hence, Hakkinen, et al., J. Natl. Cancer Inst. 44:1183-1193 (1970) and Yokota, et al., Cancer Res 41:4185-4190 (1981), have observed expression of incompatible A antigen in gastric tumors and hepatocarcinomas of blood group H or B individuals. Ernst, et al., Lab Invest 50:394-400 (1984), and Brown, et al., Int. J. Cancer 33:727-736 (1984), have observed increased expression and secretion of Lewis antigens in gastrointestinal tumors. Accumulation of abnormal fucosylgangliosides and enhancement of irregular secretion of type 2 chains has been reported in lung and colon cancers. Magnani, et al., J. Biol. Chem. 257:14365-14369 (1982); Huang, et al., Arch. Biochem. Biophys 220:381-320 (1983); Fukushi, et al. J. Exp. Med. 159:506-520 (1984). Recently, solid phase radio immunoassays which have been developed for antibodies that reach with Lewis antigens have shown increased serum levels in cancer patients, particularly adenocarcinomas of the colon, stomach, and pancreas. Koprowski, et al., Science 212: 53-54 (1981); Chia, et al., Cancer Res. 45:435-437 (1985).

It will be seen, supra, that the expression of blood group antigens by cancers associated with the gastrointestinal tract, and the colon, has been recognized in the field.

The importance of monoclonal antibodies for the diagnosis of cancer has also been recognized. Cell surface antigens, for which specific monoclonal antibodies have been prepared, are often characteristic of cancer in general, as well as specific cancer types. Given the observation that blood group antigens are expressed by cancerous cells, it is desirable to determine the presence of these antigens on cell types which do not normally produce them, or which would normally produce a different blood group antigen.

- 4 -

Hence it is an object of this invention to provide a means for detecting cancer cells, especially cancer of the colon and gastrointestinal tract, using monoclonal antibodies specific to particular blood group antigens.

It is a further object of this invention to provide panels of monoclonal antibodies which detect gastrointestinal and colonic cancer in general, and which can distinguish between different types of cancer.

How these and other objects of the invention are accomplished will become apparent from the disclosure which follows.

## BRIEF DESCRIPTION OF THE FIGURES

Figure 1 shows the structures of H, $Le^a$, $Le^b$, X, and Y blood group determinants.

Figure 2 shows expression of $Le^a$, $Le^b$, X, and Y blood group antigens on colonic tumors classified according to the localization of the lesions. The ordinate indications the percentage of individuals expressing the antigen in normal (hatched bars) and malignant (filled bars) tissues. For distal colon, $N = 35$; for proximal colon, $N = 7$; for metastases, $N = 10$.

Figure 3 shows expression of $Le^a$, $Le^b$, X, and Y blood group antigens on colonic tumors classified according to their type and degree of differentiation. The ordinate indicates the percentage of individuals expressing the antigen. 1: well-differentiated primary tumors (N = 4); 2: moderately differentiated primary tumors (N = 29); 3: poorly differentiated primary tumors (N = 6); 4: metastatic tumors (N = 10).

Figure 4 shows immunohistologic analysis of normal colonic epithelium and colonic carcinoma (frozen sections) with anti-Le[a] and anti-Y blood group antibodies. A: normal colon with anti-Le[a] (Ab174); B: colonic carcinoma with anti-Le[a] (Ab174); C: normal colon with anti-Y (AbF3); D: colonic carcinoma with anti-Y (AbF3).

Figure 5 shows immunostaining of neutral glycolipids isolated from normal and malignant tumor samples from the same individual with anti-Le[a], anti-Le[b], anti-X, and anti-Y antibodies. N = normal tissue samples; T = tumor tissue samples; S = standard slycolipids as indicated on the right of each panel.

Figure 6 shows normal colonic mucosa and carcinoma of the rectum from a blood group A patient stained with monoclonal antibodies detecting precursor type 1 chain (A and B) and A (C and D) antigens. Original magnifications: x100.

Figure 7 shows colonic mucosa and carcinoma of the rectum from a blood group A and secretor patient stained with monoclonal antibodies detecting Le[a](A and B), Le[b](C and D), X(E and F), and Y(G and H). Original manifications: x200.

Figure 8 shows sigmoid colonic mucosa (A and C) and metastatic sigmoid colon carcinoma in a regional lymph node (B and D) from a blood group A and secretor patient stained with monoclonal antibodies detecting Le$^a$ (A and B) and Y(C and D) antigens, respectively. Original manifications: x200.

## DETAILED DESCRIPTION OF PREFERRED EMBODIMENTS

The hybridoma cell lines which produce the monoclonal antibodies used in connection with this invention are on deposit at the Sloan Kettering Institute for Cancer Research, 1270 York Avenue, New York, N.Y. 10021. In addition, the hybridoma cell lines will be made available to the public via the American Type Culture Collection, Bethesda, Maryland.

Monoclonal Antibodies

Monoclonal Antibodies T-174 and T-218 were produced after immunizing mice with human colonic cancer cell lines SK-CO-10, using standard procedures as described by Kohler, et al., Nature 256:495-496 (1975), and Dippold, et al., PNAS 77: 6114-6118 (1980). Monoclonal antibodies P-12 (anti-X) and F-3 (anti-Y) have been described by Rettig, et al., Cancer Res. 45:815-821 (1985); Anger, et al., Hybridoma 1:139-147 (1982); Lloyd, et al., Immunogen 17:537-541 (1982).

Purified agglutin I from Ulex europeus, at 4mg/ml was used, to identify H-antigen. H29-36 is described in Sakamoto, et al., Ser. No. 474,415, filed March 11, 1983, indicated as allowed. Monoclonal antibody S8 has been described by Ueda, et al., PNAS 78:5122-5126 (1981); Ser. No. 297,814, filed August 31, 1981.

Blood group glycoproteins and glycolipids

Blood group-active glycoproteins were obtained from ovarian cyst fluids.


Cell Lines and serological procedures

Human tumor cell lines and their culture has been described previously by Dippold, et al., *supra*. For assay of IgG1 antibody T-174 on cell lines, the mixed hemadsorption assay (MHA) was carried out using rabbit anti-mouse Ig. Koo, et al., *J. Immunol. Methods* 23:197-201 (1978). For the IgM antibodies (T-218, P-12, F-3 and K-21) the immunoadherance assay (IA) was used. Ueda, et al., *J. Exp. Med.* 150:564-579 (1971). Monoclonal antibody-containing sera from nu/nu mice or ascites fluid were titrated starting at a dilution of $2 \times 10^2$; the results were confirmed by absorption tests with the cell line being studied.

Inhibition tests with the ovarian cyst glycoproteins Rettig, *supra* and direct ELISA assays with purified glycolipids absorbed to the wells of microtiter plates, were carried out as described previously. Lloyd, et al., *Immunogen*, *supra*.


Tissue specimens

Primary clolrectal carcinoma tissue was obtained from 42 patients undergoing surgical resection. Seven were from proximal colon (3 caecum, 4 ascending colon), one was from transverse colon and 34 were from the distal colon (7 descending, 5 sigmoid, 8 rectosigmoid, 14 rectum). Four were

SK 387-JEL/NDH

0248147

well-differentiated, 29 were moderately differentiated and 6 were poorly differentiated type carcinomas among the primary cancers. In all cases, normal mucosa distant from the tumor lesion was obtained from the same patient. Ten metastases of colon carcinoma to the liver were also analyzed. All tissue samples were frozen in isopentane solution cooled by dipping into the liquid nitrogen, and cut into serial sections from immunoperoxidase staining.

## Immunoperoxidase staining

The immunoperoxidase staining method was carried out as follows: frozen sections were thawed at room temperature for 30 minutes, and then fixed in buffered formalin for 5 minutes. The sections were incubated with 2% hydrogen peroxide for 15 minutes. After washing, monoclonal antibodies (nu/nu or ascites fluid, diluted 1:250 in 10% normal goat serum) were applied for 1 hour at room temperature. After washing 3 times with PBS, the section was incubated for 1 hour with biotinylated either horse or goat anti-mouse IgM or IgG, respectively. Sections were then incubated for 45 minutes with avidin-biotin complex, washed several times, and flooded for 15 minutes in 3-amino 9-ethylcarbazol (AEC) or 5 minutes in diaminobenzidine (DAB). Counter-staining was carried out by hemotoxylin and the specimen mounted in elbanol. MAb C-360, which is directed to antigen on melanoma cells, was used as a negative control. Staining with mAb H-26, which reacts with all epithelial cells was used as a positive control. For formalin-fixed and paraffin-embedded sections, a modification
of this technique was used.


Expression of the antigens in saliva, tissue sections and in
glycolipids from the same individuals

Samples from seven patients were analyzed for the
expression of the antigens in saliva, in colonic tissue
sections by the immunoperoxidase procedure and in glycolipids
extracted from the same specimens.

Saliva was tested by an ELISA procedure as described
previously by Sakamoto, et al., Mol. Immunol 21:1093-1098
(1984). Neutral glycolipids were isolated from the tissue
samples as follows: tissue as extracted sequentially with 30
volumes chloroform:methanol (C:M), 2:1, 1:1 and 1:2 and the
glycolipids from the combined extracts were isolated by
Florisil chromatography on acetylated samples as described by
Saito, et al., J. Lipid Res. 12:257-259 (1971); the glycolipid
fraction was then sub-fractionated into neutral and acidic
glycolipid components by DEAE- Sephadex chromatography. Yu, et
al., J. Lipid Res. 13:680-686 (1972). Reactivity of the
samples with the various antibodies was determined by
separating aliquots of the neutral glycolipid fractions by thin
layer chromatography (TLC) in chloroform:methanol:water
(60:35:8) solvent and immunostaining the reactive components as
described by Magnani, et al., Anal. Biochem. 109:399-402 (1980)
and modified by Young, et al., J. Biol. Chem. 258:4890-4894
(1983). The mAbs were used at a dilution of 1:500 and rabbit anti-mouse IgG and $^{125}$I-protein A was used as the detecting reagent.

Production and characterization of anti-$Le^a$ and anti-$Le^b$ mouse monoclonal antibodies:

Six monoclonal antibodies directed against Lewis-related antigens were used in this set of experiments. Five antibodies (T-174, T-85, T-307, T-15 and T-218) were produced from the fusion of myeloma cell NS/1 with the spleen cells from mice immunized by colon carcinoma cell lines SK-CO-10. The other antibody H-68 was derived from immunization with the colon cancer cell line HT-29. MAbs T-174, T-15, T-85, T-307 and H-68 were all inhibited by an $Le^a$ glycoprotein from ovarian cyst mucin (N-1) (Table I). None of the antibodies were inhibited by the other blood group glycoproteins tested, except that H-68 was also weakly inhibited by H and $Le^b$ glycoprotein. The anti-$Le^a$ specificity of mAbs T-174, T-15, T-85 and T-307 was confirmed by testing their ability to react with $Le^a$ glycolipid ($Le^a$-5) in direct ELISA tests and on thin layer chromatograms by immunostaining (Table I). MAb H-68 showed reactivity with $Le^a$ glycolipid in an ELISA assay but also had a low degree of reactivity with H-1 glycolipid, indicating that it has a wider specificity and recognizes a determinant shared between H, $Le^a$ and $Le^b$ structures. MAb T-218 was inhibited by $Le^b$ glycoprotein and by A and H glycoproteins (which are known to contain small amounts of $Le^b$ determinants) at considerably higher concentrations. In tests with purified glycolipids, it reacted only with Le$^b$ glycolipid (Table I). For the analysis of tissues as described infra mAb T-174 was chosen as the anti-Le$^a$ reagent and mAb T-218 was used as an anti-Le$^b$.

Blood group antigen expression on cancer cell lines

On the 155 cancer cell lines tested, Le$^a$ was found to be expressed on 7/8 cell lines from well-to moderately well-differentiated colon cancer and in 4/9 lines from poorly differentiated or metastatic colon cancers. Other cell lines tested were essentially Le$^a$-negative, except 4 lung, 1 breast, 1 ovarian and 1 B cell leukemia cell lines (Table II).

Le$^b$ expression was, in general similar to Le$^a$ expression. Thus 8/8 cell lines from well-diferentiated colon cancers and 2/9 from poorly differentiated or metastatic cancers express Le$^b$. Le$^b$ expression in lung cancers was found to be relatively higher than Le$^a$ expression (10/23). Le$^b$ was also found to be expressed in 2/11 bladder cancer cell lines, 2/11 breast cancer cell lines, 1/20 renal cancer cell lines and 2/4 ovarian cancer cell lines.

X, Y, and H-2 antigens were found to be more widely distributed in several types of cell lines. X antigen was strongly positive in colon cancer, and in breast cancer lines. X antigen was also positive in some lung cancer, bladder cancer, ovarian cancer, choriocarcinoma cell lines and in monocyte and null cell leukemias. Y antigen is expressed strongly in colon, lung, breast, and ovarian cancer cell lines.

H-2 antigen was particularly well-expressed on lung and breast cancer cell lines.

None of the melanoma or astrocytoma cell lines or cultured fibroblasts tested showed reactivity with any of the anti-blood group antibodies tested.

## Blood group antigen expression in colonic cancer and in normal colonic epithelium of the same individual

The expression of $Le^a$, $Le^b$, X and Y antigens in colonic cancer specimens and normal colonic epithelium from the same individuals was determined using the immunoperoxidase method (Table III).

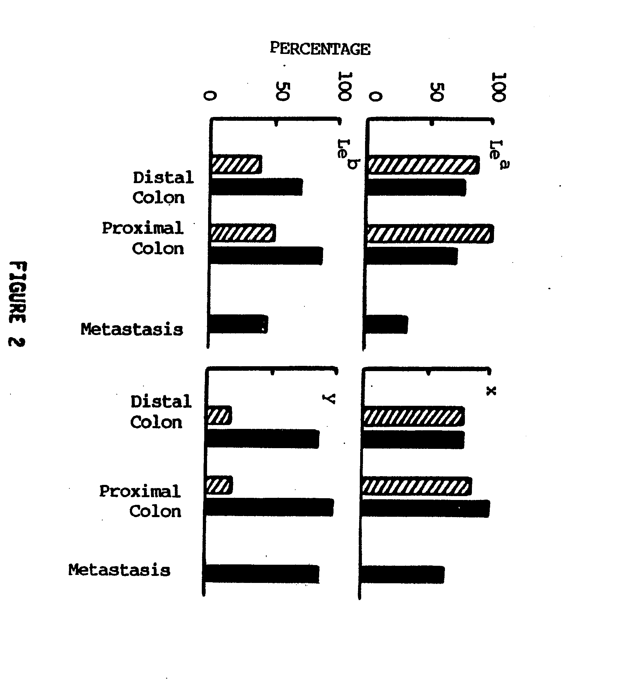

In normal mucosa from proximal (transverse and right colon and caecum) and distal colon (rectum, rectosigmoid, sigmoid, and left colon), $Le^a$ and X were expressed in most of the specimens. $Le^b$ was expressed in 4/7 proximal colon and in 14/34 distal colon specimens. Y antigen expression was similar to $Le^b$. Y was expressed in 3/8 proximal colon and in 14/34 distal colon specimens as shown in Figure 2.

In carcinoma tissues, $Le^a$ expression was found in 6/8 tumors of the proximal colon and in 26/34 tumors of the distal colon. X expression was found in 7/8 proximal colon and in 29/30 distal colon tumors. $Le^b$ was expressed in 7/8 proximal colon and in 26/34 distal colon tumors. Y was expressed in 8/8 proximal, and in 31/34 distal colon tumors. $Le^b$ and Y expression was greatly increased in carcinoma tissues as compared to their presence in the corresponding normal tissues;

in some individuals (15/42 for Le$^b$ and 22/42 for Y) the antigen was detected in the tumor samples but not in normal colonic epithelium (Table III and Figure 2).

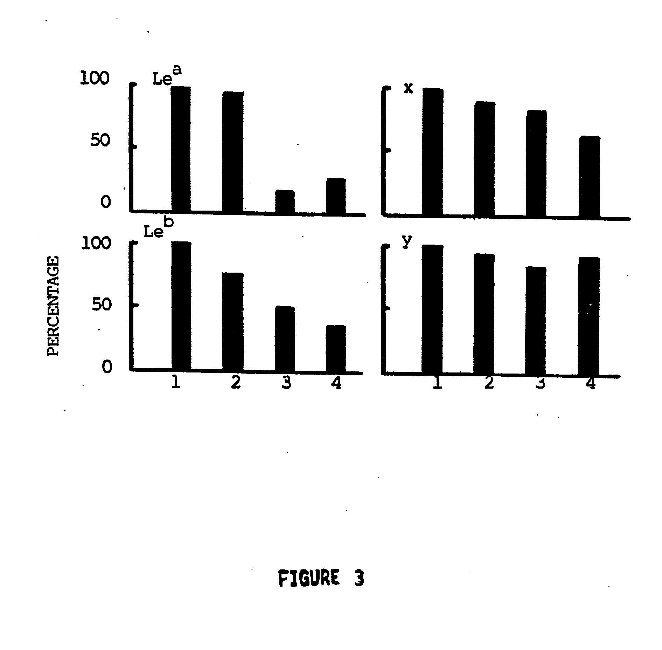

When antigen expression was correlated with the pathological grading of the tumors it was found that X and Y expression did not differ significantly in well, moderately or poorly differentiated colon carcinomas and in metastatic cancers (Figure 3). On the other hand, Le$^a$ and Le$^b$ antigen expression was decreased in poorly differentiated carcinomas and in metastatic carcinoma as compared to the well and moderately differentiated carcinomas (Figure 3).

Immunohistologic staining and glycolipid analysis of tumor and normal tissues from the same individuals and correlation with secretor status.

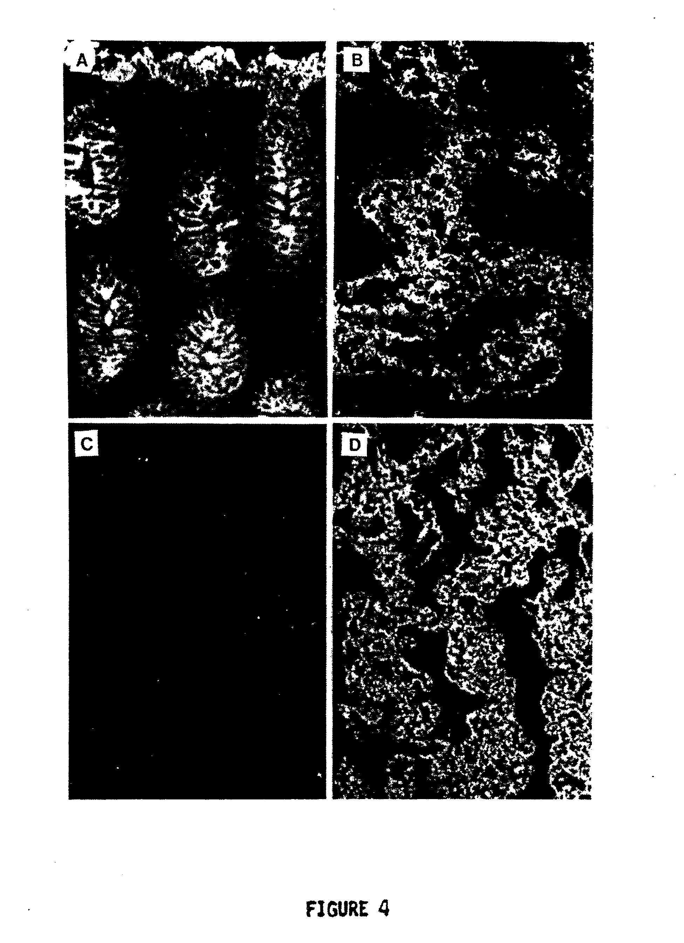

Seven patients were chosen for a more detailed analysis. Le$^a$, Le$^b$, X and Y expression in tissue sections was determined by the immunoperoxidase assays on both frozen and paraffin-embedded samples; identical results were obtained using either material (Figure 4). In addition, blood group-reactive glycolipids from five of the same specimens were examined by immunostaining of TLC plates. Saliva from the same individuals was also analyzed to determine their secretor status (Table IV).

By immunoperoxidase staining, tissue sections from all 7 patients were shown to express Le$^a$ and X (regardless of their secretor status); in 4 of these patients decreased Le$^a$

- 14 -

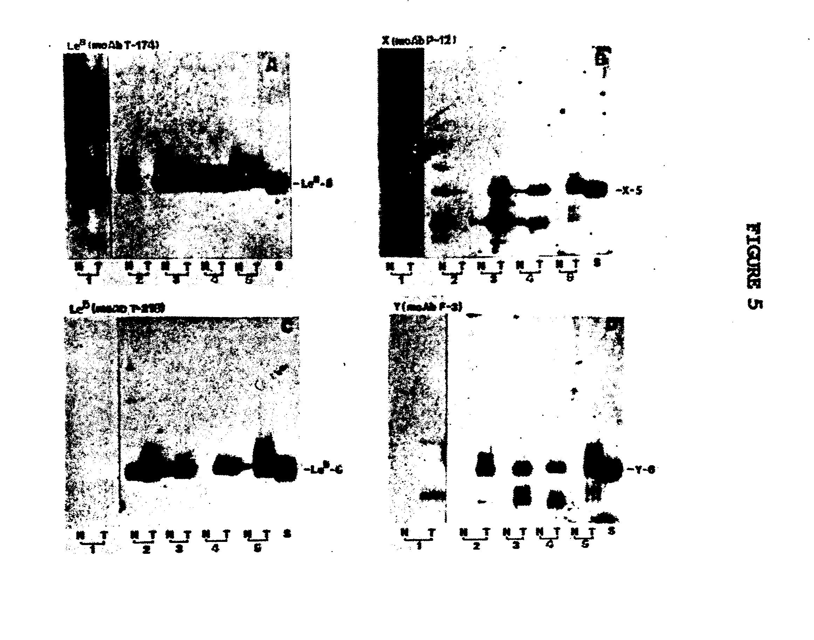

expression was observed in their tumors supra. These results were generally confirmed by examining the immunoreactivity of glycolipids extracted from the samples. Le$^a$ reactive glycolipids could be detected in all 5 normal tissue samples; the reactive glycolipid migrated with the corresponding pentaglycosylceramide in each case (Figure 5). Four of the five tumor specimens also contained Le$^a$- reactive glycolipids. The expression of X antigen was more variable: n 4 of the individuals X antigen was enhanced in the tumor whereas in one patient it was reduced (Figure 5). Interestingly, although all five samples contained the expected

-active pentaglycosyl-ceramide, they also had varying amounts of slower migrating, larger X glycolipids (Figure 5). These species may correspond to the poly-fucosylated glycolipids described by Hakomori, et al., J. Biol Chem. 259: 4672-4680 (1984).

Le$^b$ antigen was found to be weakly expressed in normal colonic tissues from 2/7 individuals examined by the immunoperoxidase method and absent in others. On the other hand, 5/7 patients showed Le$^b$ expression in the tumor specimens; Le$^b$ could not be detected in the corresponding normal tissue in three of these individuals. These results were confirmed by examining the glycolipids from the same specimens. Four of the individuals showed greatly enhanced expression of Le$^b$-reactive glycolipids in their tumor samples as compared with the normal specimens. In the case of one of the non-secretor individuals, no Le$^b$ could be detected in the normal tissue whereas the tumor sample showed strong Le$^b$ expression. In each case only one species of Le$^b$ glycolipid was detected which migrated with hexaglycosyl ceramide (Figure 5).

Y antigen was found in only one normal sample (a secretor individual) by immunoperoxidase staining of tissue sections and this sample came from an area adjacent to tumor (Table IV). Glycolipids from none of the five normal samples stained showed reactivity with anti-Y. In contrast, tissue sections from all seven tumor specimens were positive for Y antigen by immunoperoxidase staining (Figure 4). Likewise, all five tumor samples examined for glycolipids showed Y reactivity; this included the two tumors from non-secretor individuals. In additions to the hexaglycosyl-ceramide Y-6 glycolipid each sample showed the presence of a larger Y-reactive glycolipid and in the case of specimen 1 this was the only form detected (Figure 5).

In a second set of experiments, distribution of blood group antigens in the gastrointestinal tract was studied.

a)  Indirect Immunofluorescence:  Frozen tissue sections (4 to 8 microns) were cut using a cryostat. Cut sections were used unfixed or fixed for 10 minutes with either 1% formalin in PBS or cold acetone. TIssue sections were washed several times in PBS and rinsed in 2% bovine serum albumin in PBS(BSA-PBS). They were then incubated in a set chamber with primary antibodies for one hour at room temperature, the titration and appropriate dilution having been previously established.

Sections were washed with PBS and incubated for 45 minutes with secondary fluoresceinsted antibodies, also previously titrated for optimal dilution (usually 1:40 in BSA-PBS) Tissue sections were washed extensively in PBS, with turbulence created by a magnetic stirring plate, wet mounted in 90% glycerol in PBS and examined with a fluorescence microscope equipped for epifluorescence.

b) Immunoperoxidase: Formalin-fixed, paraffin-embedded sections were deparaffinized and treated for 30 minutes in 1% hydrogen peroxide in PBS in order to remove endogenous peroxidase activity (no staining was observed when 1% periodic acid was used instead of 1% hydrogen peroxide). Tissue sections were washed several times in PBS, then incubated with the appropriate supressor serum for 30 minutes, drained, and incubated with appropriately diluted primary antibody overnight at 4°C. Both peroxidase-antiperoxidase and avidin-biotin methods were used in the experiments; the procedures were similar to those previously described by Sternberger Immunochemistry (2d ed.) John Wiley & Sons Inc. (N.Y. 1979); Hsu Am. J. Pathol. 75: 734-740 (1981) et al. The secondary antibodies were horseradish peroxidase conjugated and incubated on sections for 1 hour. Sections were washed several times in PBS and rinsed with 0.05M Tris buffer, 0.1 M NaCl, at pH 8. For the final reaction diaminobenzidine (DAB) was used as chromogen, incubating tissue sections for 6 to 12 minutes with 5 mg of DAB tetrahydrochloride 100ml of tris buffer containing 100 ug of 0.3% hydrogen peroxide. Sections finally were washed with distilled water, counterstained with hemotoxylin, and mounted.

Fresh frozen tissue sections were also stained by this method. In this case, antibodies were incubated for 1 hour, and the other steps were similar to those described above for paraffin-embedded tissue sections.

c) Method for staining with lectin: Formalin-fixed, paraffin-embedded sections were prepared as above. The lectin Ulex europeus was incubated for 2 hours at room temperature, followed by rabbit anti-Ulex lectin antibody at a dilution of 1:1000 overnight at 4°C. Immunoperoxidase methods were performed as described above using biotinvlated goat anti-rabbit immunoglobulins as secondarv reagent.

d) Controls: Frozen and paraffin-embedded tissues expressing the appropriate blood antigen served for titration of the reagents as well as positive and negative controls. Negative controls included substitution of the primary antibody by another antibody of the same species and isotype, or PBS alone.

## RESULTS

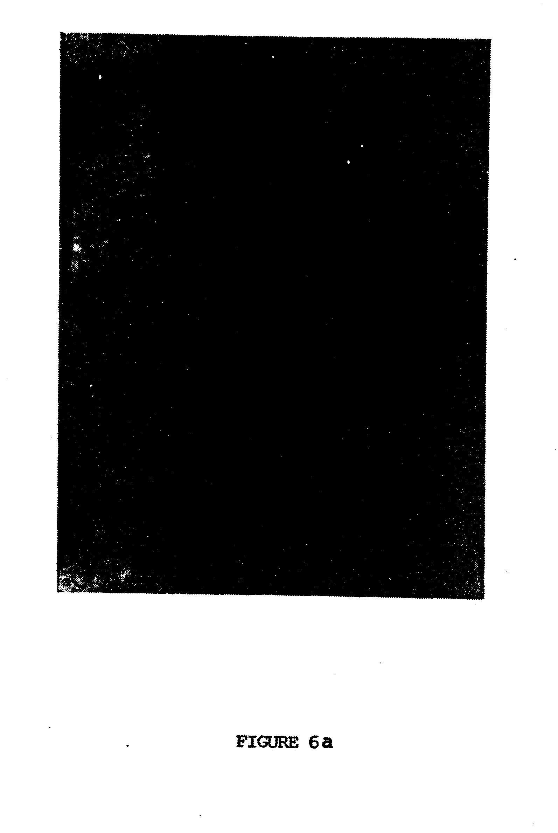

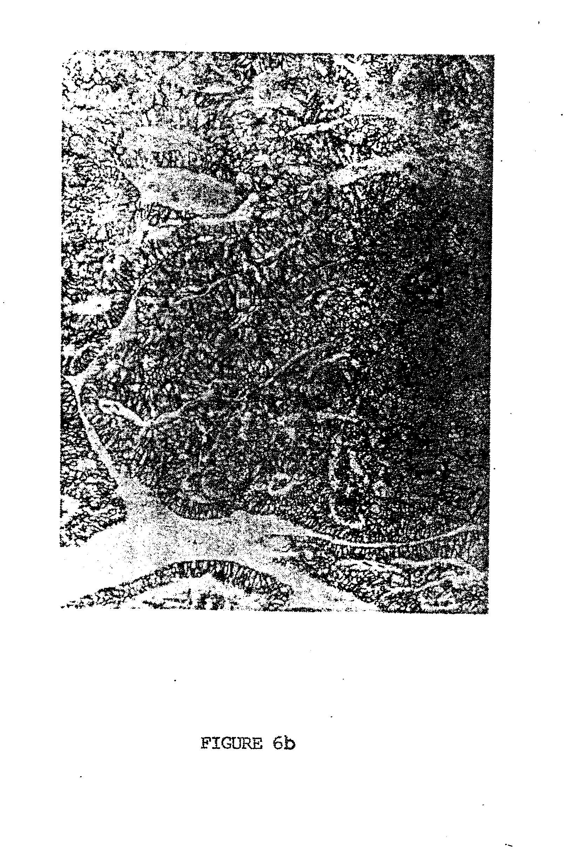

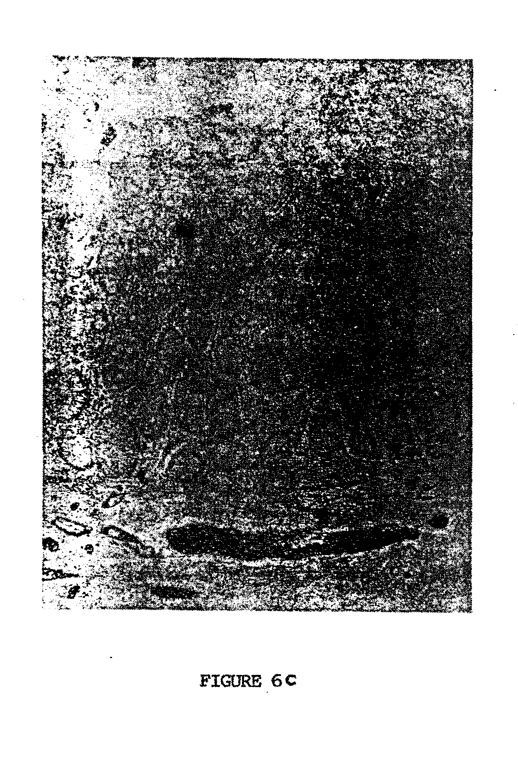

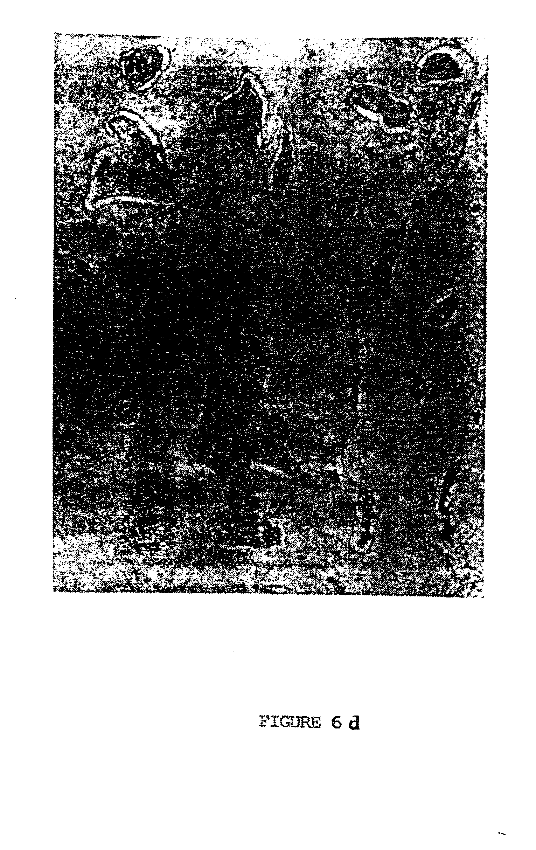

Table V summarizes the derivation of the panel of mouse mAbs, their immunoglobulin subtype, and the characteristics of the blood group antigens detected. Table VI summarizes the immunoreactivities of these antibodies on sections of normal human gastrointestinal tissues. Tables VII through IX summarize the clinical information and the immunoreactivities of

- 18 -

this panel of antibodies on sections of colorectal tumors. Figures 6, 7, and 8 illustrate the immunohistological staining patterns of these mAbs with normal human adult colonic mucosa and colorectal adenocarcinoma.

Blood group reactivities in normal human tissues.

Table VI summarizes the reactivities observed in normal human fetal and adult tissues of the gastrointestinal tract. No differences were detected between frozen tissue sections and formalin-fixed, paraffin-embedded tissue samples. Pretreatment with hydrogen peroxide was required in order to obtain immunoreactivities in paraffin-embedded sections. Frozen sections did not require pretreatment.

Precursor type 1 antigen was detected in suprabasal cells of fetal esophagus, chief and parietal cells of fetal and adult stomach, goblet and absorptive cells of fetal and adult small intestine, and brush border of absorptive cells of fetal and adult colon.

A, B, and H antigens were detected in secretions and crypt cells of fetal colon. H antigen was also found in crypts of adult colon mucosa. However, A and B antigens were not detected in normal adult epithelial cells of gastrointestinal tract.

Le$^a$ antigen was expressed by suprabasal cells of both fetal and adult esophageal mucosa. Fetal stomach epithelial cells were strongly and homogeneously stained with anti-Le$^a$ antibody; adult mucosal epithelium was almost entirely negative, and reactivity was only observed in luminal secretions and some cells deep within gastric pits. Fetal and adult small intestine goblet and absorptive cells were strongly positive for anti-Le$^a$ antibody, while Paneth cells were negative. In both fetal and adult colonic mucosa, Le$^a$ was detected in goblet and absorptive cells with a strong and homogeneous cytoplasm pattern. It was also found in colonic secretions, homogeneously along the luminal surface and patchy in the crypts.

Le$^b$ antigen was found with a patchy staining and secretory pattern on suprabasal cells of fetal and adult esophagus. Epithelial cells of both fetal and adult stomach expressed Le$^b$ antigen, mainly cells deep within gastric pits. Absorptive and goblet cells of the small intestine were heterogeneously stained by anti-Le$^b$ antibody, while Paneth and crypt cells were negative. No reactivity was detected with anti-Le$^b$ antibody in normal fetal and adult colonic mucosa, regardless of blood type or secretory status.

X antigen was detected on epithelial cells of fetal and adult esophagus with strong staining of the suprabasal cells. Both fetal and adult gastric mucosa homogenously expressed Le$^a$ determinant. Crypt cells of both fetal and adult small intestine and colon were positive for anti-X antibody; goblet and absorptive cells were unreactive in the small intestine and weak or unreactive in the colon.

Y antigen was strongly and homogenously expressed by epithelial cells of both fetal and adult esophageal and gastric mucosa. In the small intestine, anti-Y reactivity was detected in crypt and Paneth cells, while goblet and absorptive cells were unreactive. Y antigen was not detected on epithelial cells of fetal and adult colonic mucosa, regardless of blood type or secretory status.

Blood group reactivities in human colorectal tumors. Table VII summarizes patient clinical information, including the patient's age, sex, blood group type, location of the primary carcinoma, metastatic tumor if present, and Dukes' stage. Table VIII summarizes immunoreactivities observed in the normal colonic mucosa distant from the tumor, compared with the primary and metastatic carcinoma. Table IX summarizes the findings in the six patients in whom secretory status was established, and correlates blood group type, secretory status, and the reactivities seen in erythrocytes, endothelial cells, normal colonic epithelium, tumor cells, and luminal secretions.

There were no differences in immunoreactivity attributable to different blood group types or secretory status of the cases analyzed.

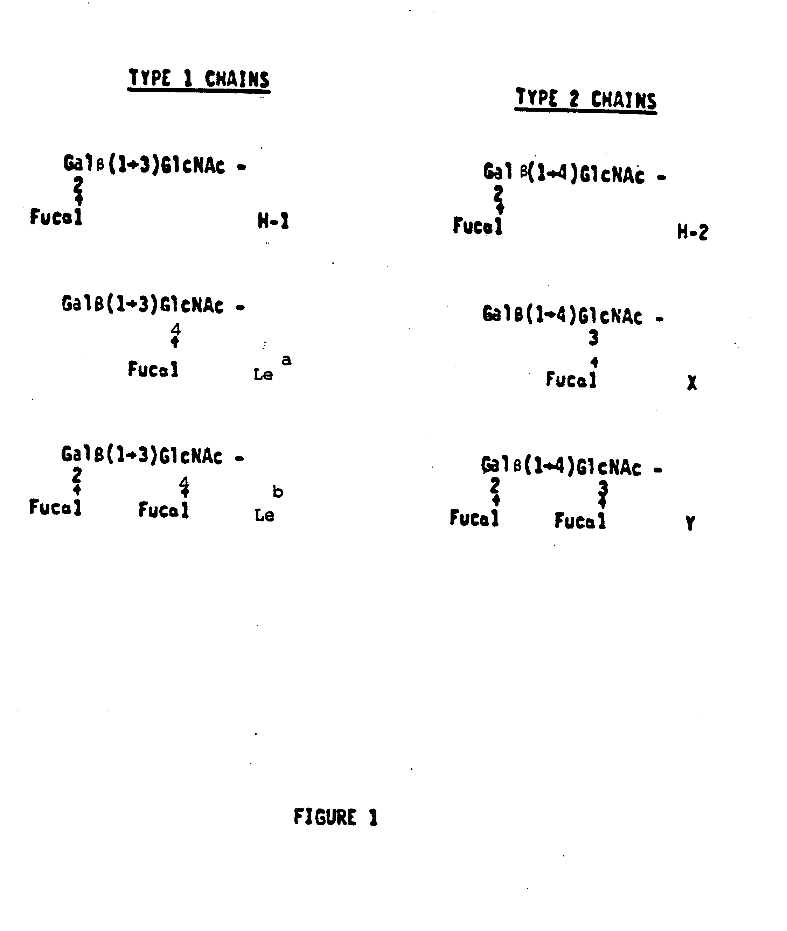

Precursor type 1 chain antigen was detected in only occasional cells of the normal colonic mucosa, more frequently in the luminal cells. The staining was mainly in the brush border. When it was present the pattern of staining in cytoplasm was punctate and heterogeneous. In several cases no staining was identified either in epithelial cells or luminal secretions (Figure 1). Erythrocytes, endothelial cells, inflammatory cells and mesenchymal cells were negative.

In two cases, both Dukes C state carcinomas, tumor cells were homogeneously stained for the precursor type 1 chain antigen; the majority of tumors showed patchy staining (Figure 6B). Only three of twenty primary and one of fourteen metastatic tumors were unreactive for mAb K21 (Table VIII).

H antigen reactivity was seen on erythrocytes, endothelial cells and in luminal secretions of normal colonic mucosa, mainly in the crypts. No reactivity was observed in the cytoplasm or cell membrane of normal colonic epithelial cells, regardless of blood type or secretory status.

Cytoplasmic immunostaining was present, but heterogeneous, in nineteen of the twenty primary tumors. Three of fourteen metastases demonstrated homogeneous staining in tumor cells; staining was hetrogeneous in ten of the eleven remaining metastic tumors and absent in one (Table VIII).

A and B blood group antigens were found on erythrocytes and endothelial cells of corresponding blood type patients. No reactivity was observed in epithelial cells of normal colonic mucosa, regardless of blood type or secretory status (Figure 6C). Only in three cases was weak reactivity found in secretions of normal mucosa near the tumor.

Four of the seven blood group A patients and the one AB patient showed immunostaining of tumors for A antigen, mainly in luminal secretions, and occasionally in the cytoplasm of a few tumor cells (Figure 6D). The AB patient also stained for B

SK 387-JEL/NDH

0248147

antigen in tumor, but only one of four B patients had tumor staining with anti-B antibody. Anomalous expression of these antigens in incompatible blood type patients was not detected (see Tables VII and VIII).

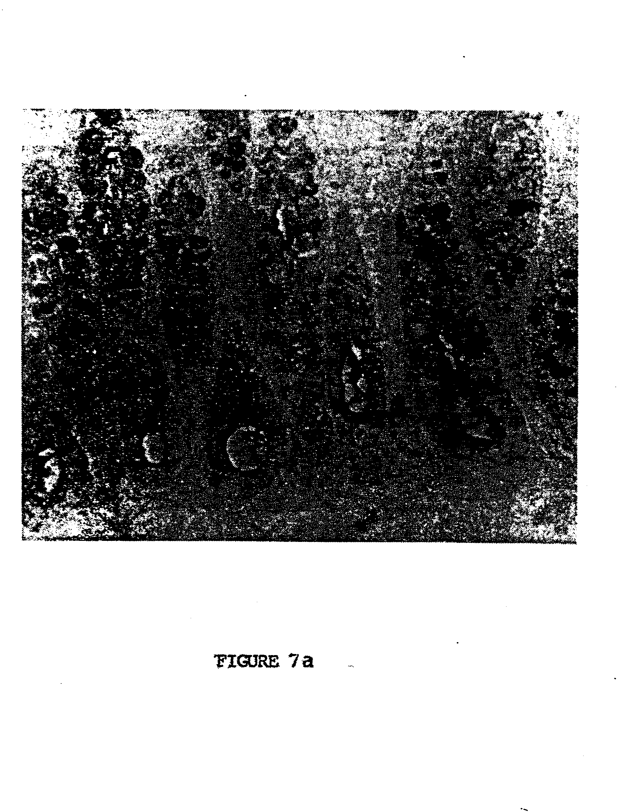

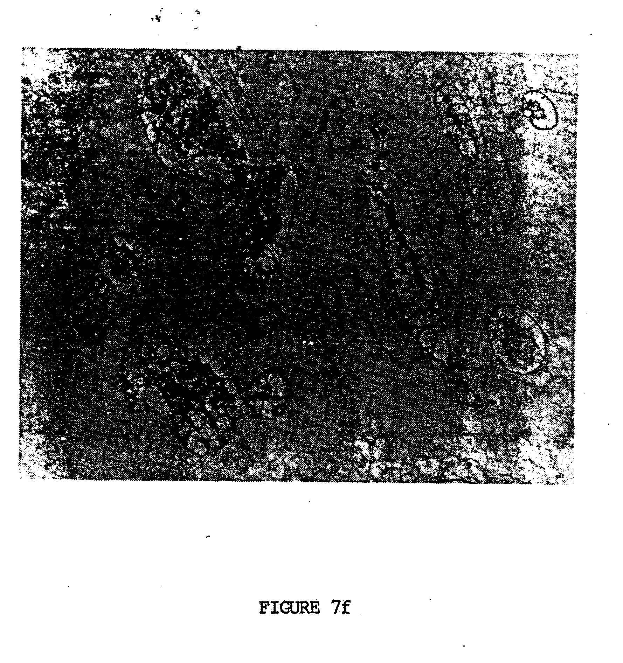

Lewis[a] antigen was expressed in the normal colonic mucosa of all but one of the cases studied, with increased reactivity in the luminal side (Figure 7A). The pattern of staining was cytoplasmic, diffuse, and homogeneous. Erythrocytes and endothelial cells were unreactive, regardless of blood type and secretory status.

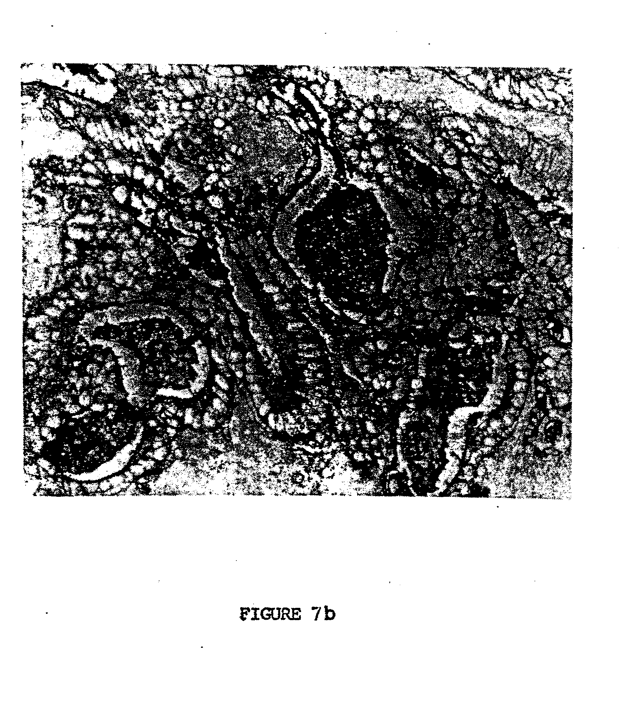

Ten of twenty primary tumors and seven of fourteen metastases showed strong immunostaining of tumor cells, either homogeneous or heterogeneous, and staining of luminal secretions (Figure 7B). Staining was present though not as strong in seven primary and three metastatic tumors. Only three primary tumors were negative (15% of the cases), while in four metastatic lesions Le[a] was not detected (28.5% of the cases).

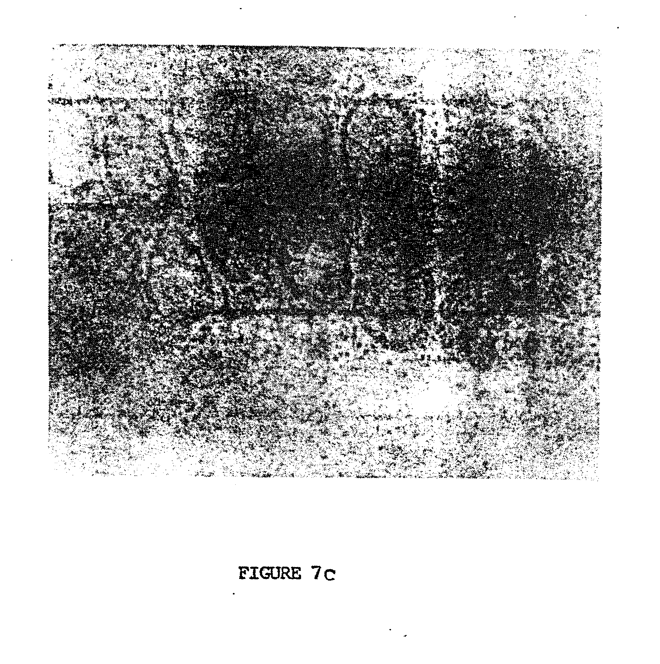

Lewis[b] antigen was expressed by only occasional cells located in the luminal epithelium of only 3 of the 20 cases studied, and in some luminal secretions of those cases. The majority of the specimens were unreactive (Table VIII) (Figure 7C). Erythrocytes, endothelial and mesenchymal cells did not stain with antibody T218, regardless of secretory status.

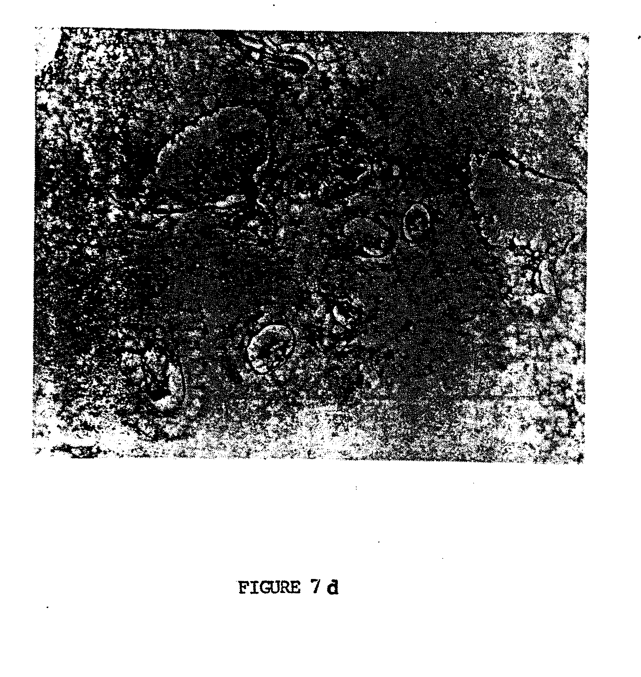

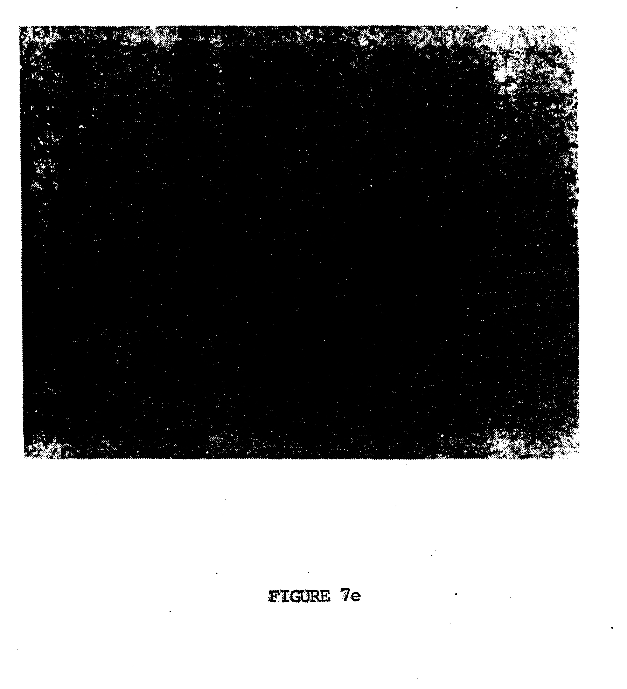

In seventeen of the twenty tumors analyzed there was intense homogeneous or heterogeneous immunostaining, including both secretor and non-secretory cases (Tables VIII and IX) (Figure 7D). Only two metastatic tumors were negative for $Le^b$ (14.2% of the cases).

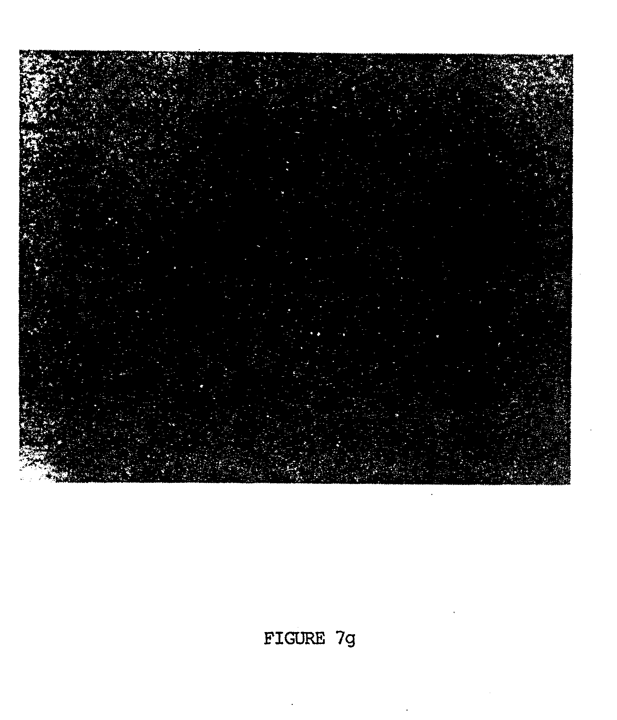

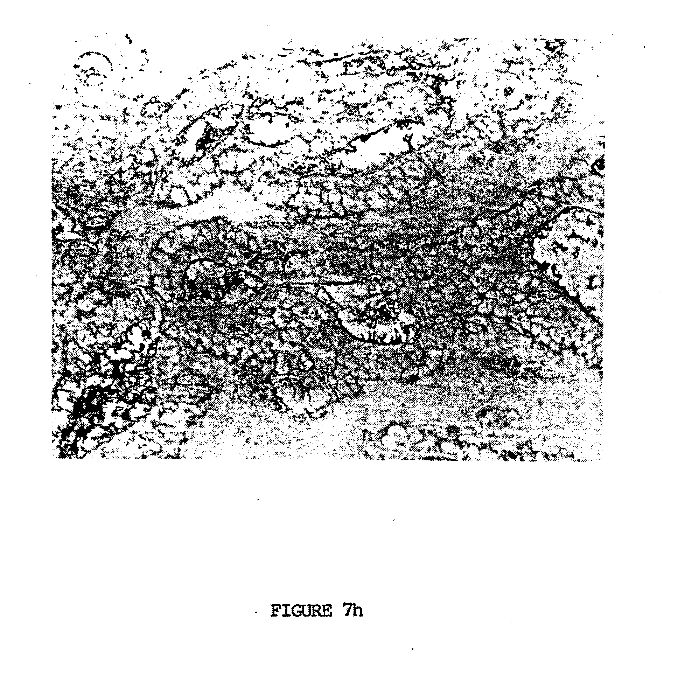

X antigen was expressed by the crypt cells of the colonic mucosa in fifteen of the twenty cases studied. There was reactivity in both cytoplasm of the epithelial cells and luminal secretion (Figure 7). Erythrocytes and endothelial cells were unreactive, but polymorphonuclear leukocytes were strongly stained with mAb P12.

There was homogeneous or heterogeneous expression of X antigen in all twenty tumors studied, and in the majority of cases the pattern of reactivity was that of diffuse, homogeneous cytoplasmic staining (Table 8) (Figure 7). Increased immunostaining was observed in invasive and metastatic tumors.

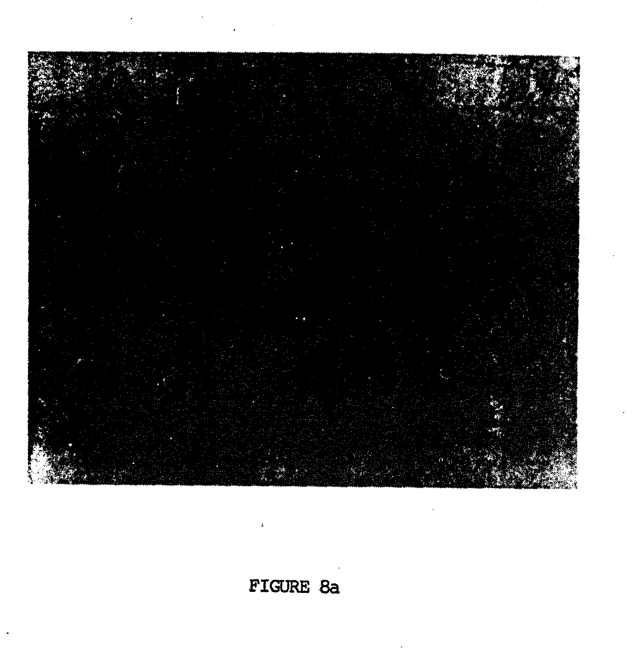

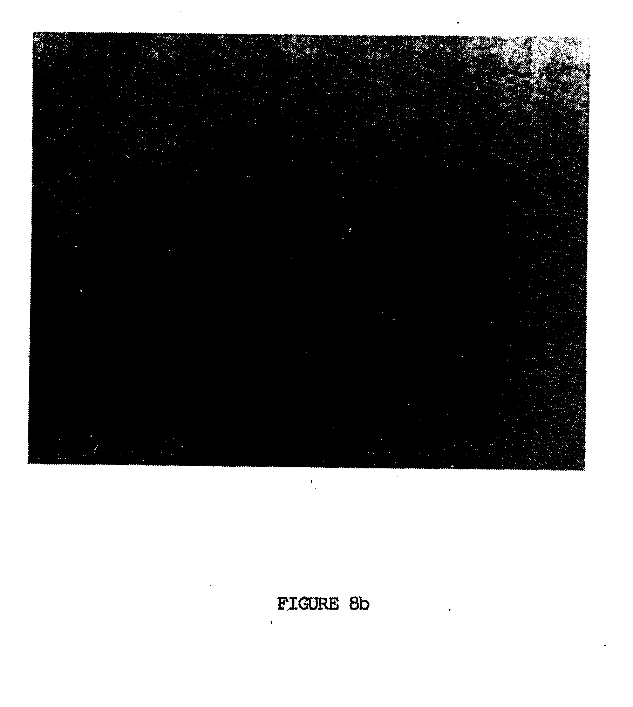

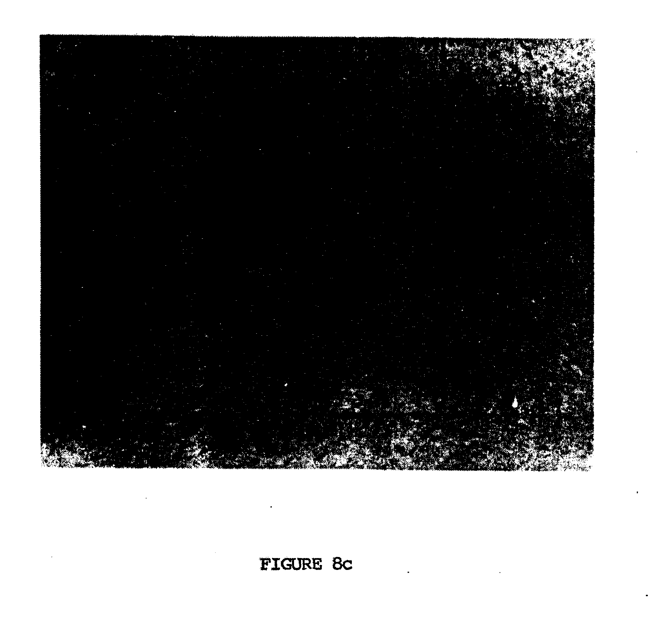

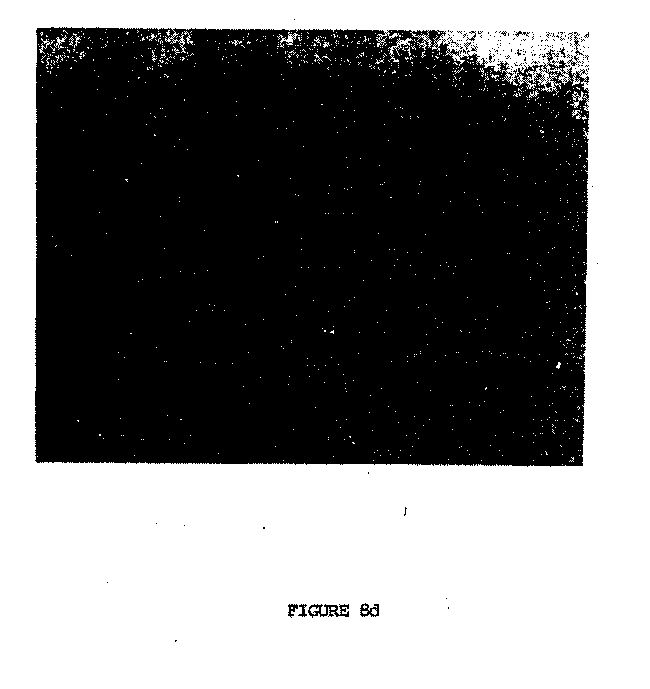

Y antigen as identified by the mAb F3 was found to react with erythocytes and endothelial cells of blood group type H specimens. It was unreactive with the epithelial cells of the normal colonic mucosa in all the cases analyzed (Table 8) (Figure 7G). In contrast, Y antigen expression was seen in all tumors, regardless of blood type and secretory status. The pattern of staining was cytoplasmic and heterogeneous in the majority of invasive and metastatic tumors studies, with strongest staining in secretaions. In several cases, when $Le^a$ was negative in the tumor (Fgure 8), Y was expressed with strong reactivity (Figure 8D).

In the first set of experiments, expression of $Le^a$, $Le^b$, X and Y antigens was determined on 165 cultured tumor and normal

- 24 -

cells lines. Colon carcinoma and normal colonic epithelium from 42 individuals was studied for the expression of the same antigens by immunoperoxidase staining of frozen sections. In seven individuals, both frozen and paraffin sections were examined and glycolipids extracted from five of the same samples were analyzed. In the smaller group, the secretor status of the individuals was determined independently by examining their saliva.

The data on cultured cells showed that epithelial cancer cell lines, particularly those originating from endoderm- or ectoderm-derived tissues (colon, lung and breast), are strong expressors of blood group antigens. $Le^a$ and $Le^b$ tended to be more widely expressed on colonic cancer cell lines than on the other cell types. X, Y, and H-2 antigens were found to be strongly expressed on the majority of epithelial cancer cell lines tested. It is interesting that no particular pattern of co-expression of the various specificities was observed. Also, the concomitant expression of $Le^a$, $Le^b$, X, and Y observed on some cell lines is a reflection of the situation in fresh tumor samples (Tables III and IV). FInally, the lack of blood group antigens on tumors of neuroectoderm-derived tissues (melanomas, astrocytomas, and neuroblastomas) is a significant finding. The only exception to this observation is the presence of X antigen on 3/5 neuroblastoma cell lines.

In normal colonic mucosa, $Le^a$ and X are almost ubiquitously expressed regardless of the secretor status of the individual. Thus, expression of $Le^a$ and X antigens was found

0248147

in nearly 90% of the specimens examined by immunoperoxidase and this was confirmed by the blycolipid analysis. It is evident that although Le$\underline{b}^A$ and Y and not Le$^a$ and X are expressed in saliva of classical secretors, Le$^a$ and X are expressed in normal colon regardless of the secretor phenotype of the individual. In tumor tissues retention of Le$^a$ disappeared in a considerable number of specimens. In colorectal tissues, Le$^a$ might be an important "marker" for the cells to be recognized as colon cells.

Le$^b$ is not always expressed in normal colonic mucosa (even in secretors), but it is expressed in a high proportion of carcinoma. Ernst et al., in Lab Invest. 50:394-400 (1984), recently reported similar findings although these investigators found a lower expression in normal distal colon than reported here. Also, Siddiqui, et al., J. Biol Chem. 253:2168-2175 (1978), previously isolated an Le$^b$-active glycolipid from colonic cancer but not normal tissue. Immunostaining of glycolipids extracted from the carcinoma of a non-secretor patient demonstrated the expression of Le$^b$, whereas the normal counterpart was completely negative for Le$^b$ reactivity (Figure 5). These findings suggest that the H gene in tissues of non-secretors is not absent but suppresses or inactive. It is possible that cancer transformation leads to the induction of 1 2 fucosyl transferase and to the expression of Le$^b$ antigen and Y antigen in the tumor of non-secretor patients.

Most intriguing is the expression of Y antigen in tumors. Y expression is very low or absent in normal colonic epithelium, but is strongly expressed in all carcinoma tissues examined by immunoperoxidase staining. Moreover, in at least one tumor section it is noticed that the more invasive part of the tumor expressed much more Y antigen than did a portion which was morphologicall comparatively normal and non-invading. Glycoplipid analysis of 5 samples from patients also showed that Y antigen is absent in normal colonic epithelium but strongly expressed in all carcinoma specimens. The detection of Y antigen in the tumors of non-secretors is a significant finding, although this and the observation of Le$^b$ in the same tumors needs to be confirmed on a larger number of samples. Even in secretors, Y antigen serves as a tumor antigen for colonic tumors as the normal colon (particularly the distal colon) is Y-negative in most individuals. In secretors, however, Y antigen is expressed elsewhere in the body in epithelia and secretions, although expression in A, AB, and B individuals may be minimal. The expression of Y antigen in the colonic tumors of non-secretors is more significant as the expression of Y antigen elsewhere in the body in such individuals is minimal or absent. It has previously been demonstrated that the synthesis of y antigen in saliva is strictly correlated with secretor status, i.e., none of the non-secretors examined expressed Y antigen whereas 18/18 secretors did Sakamoto, et al., Mol. Immunol 21:1093-1098 (1974). Whether or not occasional tissues elsewhere in the body of non-secretors can synthesize Y antigen needs to be examined further. In a recent study, Brown, et al., Int. J.

0248147

Cancer 33:727-736 (1984), demonstrated Y antigen in exocrine acini of the pancreas, in ducts of the salivary gland, as well as in the crypts of normal colon of non-secretors. These workers also demonstrated the enhanced expression of Y antigen in colonic tumors although they did not present data on the secretor status of these patients. The precise specificities of the anti-Y reagents used in such studies is clearly important. Although the F-3 antibody used in this study and the C14/1/46/10 antibody used by Brown et al., Hybridoma 1:139-147 (1982), whereas AbC14 does not, Brown, et al., Biosci Rev 3:163-170 (1983). Further, as just discussed, AbC14 stains normal colonic crypts whereas mAb F3 does not. Similar considerations apply to Le$^b$ antigen. The expression of Le$^b$ in normal tissues, particularly in non-secretors, is not completely known. To the extent that it has been examined, e.g., in saliva–Sakamoto et al., Supra, gastric mucosa and duodenum Lemieux, et al., Biochem 20:199-207 (1981) and urothelium Juhl, et al., J. Histochem Cytochem 33:309-315 (1985) epithelia of normal non-secretors express no or very little Le$^b$ antigen. Thus, using suitable reagents, the expression of Y and Le$^b$ antigens in colonic tumors of non-secretors clearly offers opportunities for the use of anti-Y and Le$^b$ antibodies in diagnosis and therapy in such individuals, particularly in A and B patients who lack or have low levels of these antigen on their red cells, Anger, et al., supra.

Another aspect of Y antigen expression that needs to be further explored is the nature of the glycolipid in colonic tumors expressing Y specificity. Thus, in all specimens examined, a major reactive species was much larger then the simplest Y glycolipid (difucosyl-N-neohexaosyl-ceramide) and in one specimen it was the only reactive species. WHether or not this larger form represent tri-. or more highly fucosylated species analogous to the di -and tri- fucosylated X glycolipids which as shown to accumulate in colonic and liver cancers remains to be determined.

The second set of experiments represents the first immunohistologic analysis of the normal human human gastrointestinal tract and colorectal carcinomas using the lectin <u>Ulex europeus</u> with specificity for H antigen and a panel of mouse monoclonal antibodies that detect precursor type 1 chain, A, B, $Le^a$, $Le^b$, X, and Y blood group antigens. This panel of reagents has been used in these experiments with consecutive tissue sections of normal and tumor samples of individuals with known blood type and in some instances known secretory status.

Precursor type 1 chain antigen is detected in occasional cells located towards the luminal side of the colonic mucosa. Enhancement of immunostaining was found in fetal tissues. This is also the case for the expression of this antigen in the urinary tract, where it was found in fetal but not adult urothelium regardless of blood type. The precursor molecule is the backbone structure for synthesis of other blood group related antigens. That we were able to detect this structure
in just occasional cells may indicate that the free precursor
molecule is readily  fucosylated or sialylated to form other
blood group specificities. Since these other antigens have a
different conformation, it is assumed that epitope detected by
mAb K21 undergoes either conformational change or is masked.
We observed increased expression of the precursor type 1 chain
in eighteen of the twenty colon carcinomas studied. Increased
expression of blood group precursor antigens was previously
reported to occur in epithelial tumors and their derived cell
lines Feizi et al., Lancet 2:391-393 (1975). More recently,
accumulation of precursor type 1 chain has been reported to
occur in human teratocarcinoma and renal cell carcinoma Rettig
et al., Cancer Res. 45:815-821 (1985). This phenomenon may be
due to the block of A or B transferases, which in turn may
result in an accumulation of the precursor molecule, and while
proposed herein, the inventors do not bend themselves to this
hypothesis.

A, B, and H antigens have been reported as constitutive
antigens present in fetal and adult epithelial cells of the
urinary tract (see, e.g., Szulman, J. Exp. Med. 119:503-515
(1964); Deal, et al., J. Urol 133:513-516 (1985). Their
expression in oral epithelium is similar, with antigens present
in both fetal and adult mucosa Vedtofte et al., Differentiation
27:221-228 (1984). In the case of the gastrointestinal tract,
however, the expression of A, B, and H antigens in epithelial
cells is related to differentiation stages of fetal

development. These antigens are expressed in early fetal life, repressed in late fetal development, and absent in adult gastrointestinal tissues Szulman supra; Watanabe et al., J. Exp. Med. 144:644-653 (1976).

In these studies it was observed that normal adult colonic mucosa may express H antigen in occasional crypt cells and secretions located in those crypts. This applies to normal colonic mucosa of control samples as well as the normal mucosa distant from colorectal tumors. A and B antigens have not been detected in epithelial cells of the normal colonic mucosa in central specimens. However, normal colonic mucosa near to colorectal tumors in two of eight type A individuals and one of five type B individuals showed immunoreactivity with anti-A and anti-B antibodies, respectively, in luminalsecretions, mainly in the crypts. No immunostaining was detected in colonic epithelial cells in those cases. The majority of tumors (nineteen of twenty) expressed H antigen in luminal secretions and in the cytoplasm of tumor cells. The pattern of this immunostaining was heterogeneous and weak. A, B, and AB individuals also presented patchy staining for A and/or B antigens in the cytoplasm of tumor cells and luminal secretions. In compatible blood group antigen expression was not observed in this study.

A, B, and H antigens have been reported to undergo modulation during malignant cellular transformation. While they detected with homogeneous staining in normal urothelium, Bergman, et al., J. Mrol 119:49-53 (1978); Limas, et al.,

Cancer 49:2476-2480 (1982), urothelial carcinoma and carcinoma "in situ" of the urinary bladder are reported to lack expression of ABH blood group antigens; and it is postulated that histologically benign urothelium in patients with bladder cancer may present with antigenically abnormal epithelial cells Coon, et. al., Cancer 56:797-804 (1985). If this is true, markers for the detection of pre-cancerous lesions and for following populations at risk of urinary bladder cancer are available in the form of A, B, and H antigen transformation. If deletion of ABH blood group antigens is due to a block in their respective transferases, leading to the accumulation of precursor and other blood group related antigens, this would explain these findings.

Incompatible expression of A-like antigen has been reported to occur in gastric carcinomas of H patients. See, e.g., J. Natl. Cancer Inst. 44:1183-1193 (1970); (Hakkinen, Hattori et al., Biochim. Biophys. Acta 666:361-369 (1981)., and also in colorectal carcinomas (Breimer et al., Cancer Res. 40: 397-908 (1980) and hepatocarcinomas (Yokota, et. al., Cancer Res. 41:4185-4190 (1981), of H or B patients. The chemical basis for the appearance of incompatible blood group specificities may be neosynthesis induced by activation of sialyltransferases. These enzymes are encoded by genes that are repressed in normal cells, and may become activated during transformation.

Lewis blood group antigens presented distinct patterns of expression within different tissues of the gastrointestinal tract and different epithelial cells, regardless of blood type and secretory status of the individual (Ernst et al., supra; Combs et al., J. Histochem. Cytochem 32:982-988 (1984). No differences have been noticed in normal fetal compared with adult colonic mucosa. The majority of carcinomas studies expressed $Le^a$. $Le^a$ antigen also has been found in colonic mucosa near to colorectal tumors. In this respect, it may be regarded as a differentiation antigen of the epithelial cells of the colon. Enhancement of the expression $Le^a$ has been reported by others in tissue sections of colorectal tumors (Ernst et al., supra and in colon tumor cell lines (Blaszczynk et al., PNAS 82: 3552-3556 (1985). However, heterogeneity and absence of this antigen in some undifferentiated and metastatic tumors has been observed. Loss of $Le^a$ and/or neosynthesis of sialosyl-$Le^a$ antigen in these cases may indicate dedifferentiation. Significant plasma levels of $Le^a$ antigen have been reported in patients with gastrointestinal tumors (Koprowski et al., supra; Magnani et al., supra, as well as pancreatic cancer (Falk et al., Biochim. Biophys. Res. Comm. 100: 383-391 (1983).

$Le^b$ has been detected on epithelial cells of the gastric mucosa, pancreas, kidney and urinary bladder (Ernst et al., supra; Juhl., supra. It is found in occasional epithelial cells of proximal colon, while distal colon and rectum do not express this antigen (Ernst et al.). The majority of normal colonic mucosa samples taken close to tumors studied in the present analysis, regardless of location in the colon, showed

- 33 -

no reactivity with mAb T-218, which detects $Le^b$ antigen. Nevertheless, enhancement of this antigen was detected in 90% of the colorectal carcinomas studied, independently of tumor location, blood type and secretory status of the individual. Increased expression of $Le^b$ has been reported in gastric and pancreatic tumors (Ernst et al., supra), as well as colorectal cancer ad 'colon tumor cell lines. Neosynthesis of $Le^b$ in tumors of non-secretor patients adds further evidence of the probable activation of an alpha 1,2 fucosyltransferase. It also demonstrates that the Hh and Se genes are not absent, but suppressed in non-secretor individuals. Both events, enhancement of enzymes and gene reactivation, seem to appear consequent to the process of cellular transformation.

Antibodies detecting X specificities have been reported to be positive in certain normal epithelial cells, such as parietal cells of the stomach and Paneth cells of small intestine [Fukushi et al., J. Exp. Med. 159:506-520 (1984)], crypt cells of colon and granulocytes (Rettig et al., supra), epithelial cells of the proximal tubules of kidney and luminal urothelial cells. The antigen detected by P12 mAb (Lewis X blood group antigen or lacto-N-fucopentaose III structure) has also been referred to as SSEA-1 antigen [Solter et al., PNAS 75:5565-5569 (1981)], and reported to have a tissue distribution similar to the X blood group related antigen [Fox et al., Cancer Res. 43:669-678 (1983)]. This antigen is expressed in gastrointestinal tumors (Fukushi et al., supra), lung cancers [Holmes et al., J. Biol Chem 260:7619-7627 (1985)], and secreted to significant levels in the serum of cancer patients (Chia et al., supra). X antigen expression has been detected in all colorectal carcinomas studied. Because the antigen is found in crypt cells, which represent the proliferative compartment of the colonic mucosa, and strong homogeneous immunostaining has been observed in most metastases and invasive cancer cells, it may be that X antigen correlates with cellular proliferation and could be a marker for aggressiveness of certain epithelial tumors.

Y antigen has been detected on epithelial cells of the colonic mucosa of normal control samples, and not on colonic mucosa either distant or close to colorectal tumors. Nevertheless, expression of Y antigen has been found on all colorectal carcinomas in this study, and enhancement of the Y antigen has been reported recently in other studies of tumors of the large bowel (Brown et al., supra). However, different antibodies detecting distinct epitopes may represent with different tissues distributions. In the present analysis mAb F3, has been used, which recognizes an epitope on Y antigen that is present in erythrocytes of H individuals. As discussed for $Le^b$ antigen, Y specificity has been detected in all tumors, independently of blood type and secretory status of the individuals. Again this implies enhancement of a fucosyltransferase and activation of Hh and Se genes.

Epithelium may be classified in two groups, according to ABH expression in normal tissues and modulation in tumors: a) constitutional expression of ABH in fetal and adult cells

SK 387-JEL/NDH

0248147

(e.g., epithelial cells of urinary tract), and b) developmental modulation of ABH from positive to negative during fetal development, with expression lost in adult cells (e.g., epithelial cells of gastrointestinal tract). In the first group, tumor cells lose expression of ABH antigens. The second group shows neosynthesis of ABH specificities that appear in tumor cells, a phenomenon that may be considered oncofetal and potentially a marker of transformation.

Deletion of A and B antigens observed in certain tumors may be due to a block of A and B transferases, which in turn may explain the accumulation of precursor molecules present in such cancers.

Modulation of Lewis antigens observed in certain tumors may be related to activation of suppressed genes (e.g., Hh and Se), and enhancement of fucosyl transferases. These changes would be responsible for neosynthesis and accumulation of these molecules in such cancers. Activation of these enzymes appears to be under genetic control. It is conceivable, as previously pointed by, e.g., Hakomori, Cancer Res. 45:2405-2414 (1985); and Marcus, Mol. Immunol. 21:1083-1091 (1984), that such regulatory events may involve oncogenes. Enhancement of GD3 has been reported recently in cells transfected by adenoviruses [Nakamur et al., J. Biochem. (Tokyo) 96:1471-1480 (1984)]. Phosphorylation of these enzymes is needed in order to obtain active transferases (fucosyltransferase and syalyltransferase) which will act upon their substrates to produce molecules such as Lewis and sialosyl Lewis antigens, difucosylgangliosides and trifucosylganliosides. The monophosphate molecules that are released then may interact with membrane cyclic nuclioside monophosphate and diphosphate pumps. These have been reported to be closely related to the p21$^{ras}$ proto-oncogene product [Toda et al., Cell 40:27-36 (1985)].

The fact that epithelial tumors have increased expression of Lewis antigens, especially Y determinant, and that such antigens are secreted in body fluids make them candidates for detection and monitoring of cancer. For certain of these antigens, including the Y molecule, in which normal tissue distribution is restricted, mAbs directed against specific epitopes can be seen to be of singular importance in the diagnosis of cancer.

The terms and expressions which have been employed are used as terms of description and not of limitation, and there is no intention in the use of such terms and expressions of excluding any equivalents of the features shown and described or portions thereof, it being recognized that various modifications are possible within the scope of the invention.

**TABLE 1. CHARACTERIZATION OF MONOCLONAL ANTIBODIES DETECTING LEWIS ANTIGENS**

| Monoclonal Antibodies | T-174 | T-15 | T-85 | T-307 | H-68 | T-218 |
|---|---|---|---|---|---|---|
| Immunoglobulin class | $\gamma$-1 | $\mu$ | $\gamma$-1 | $\mu$ | $\gamma$-1 | $\mu$ |
| **Inhibition assays [a]** | | Minimum amount for inhibition ($\mu$g/ml) | | | | |
| **Inhibitors:** | | | | | | |
| H glycoprotein | >400 | >400 | >400 | >400 | 25 | 25 |
| H Le[b] glycoprotein | 400 | 400 | >400 | 400 | 25 | 0.375 |
| A+H Hog mucin | >400 | >400 | >400 | >400 | >400 | >400 |
| A glycoprotein | 400 | 400 | 400 | 400 | >400 | 6 |
| Le[a] glycoprotein | 0.37 | 0.37 | 6 | 1.5 | 1.5 | 400 |
| H Hog mucin | >400 | >400 | >400 | >400 | >400 | >400 |
| Precursor glycoprotein | >400 | >400 | >400 | >400 | >400 | >400 |
| **ELISA with purified glycolipids [b]** | Le[a] | Le[a] | Le[a] | Le[a] | H,Le[a] | Le[b] |
| **TLC immunostaining [c]** | Le[a] | Le[a] | Le[a] | Le[a] | n.d. | Le[b] |

Table 1 - footnote

a  Inhibition of reactivity with cell lines determined with IA assay.

b  Glycolipids tested were: Galβ(1→3)GlcNAcβ(1→3)Galβ(1→4)Glc – Cer (LNT); Galβ(1→4)GlcNAc β(1→3)Galβ(1→4)Glc – Cer (LNneoT); Fucα(1→2)Galβ(1→3)GlcNAcβ(1→3)Gal β(1→4)Glc – Cer (H-5-1); Fucα(1→2)Galβ(1→4)GlcNAcβ(1→3)Galβ(1→4)Glc – Cer (H-5-2); Galβ(1→4)[ Fucα(1→3)]GlcNAcβ(1→3)Galβ(1→4)Glc – Cer (Le[a]–5); Galβ(1→4)[Fuc α (1→3)]GlcNAcβ(1→3)Gal – Cer (X–5); Fucα(1→2)Galβ(1→3)[Fucα (1→4)]GlcNAcβ(1→3)Gal β(1→4)Glc – Cer (Le[b]–6); Fucα(1→2)Galβ(1→4)[ Fucα(1→3)]GlcNAcβ(1→3)Galβ(1→4)Glc –Cer (Y-6)

c  Determined on the total neutral glycolipids extracted from HT-29 colon cancer cell lines by method of Magnani et al., (25).

| | | | | | |
|---|---|---|---|---|---|
| | >400 | >400 | >400 | >400 | >400 | >400 |
| | 400 | | >400 | <400 | | |
| | >400 | >400 | >400 | >400 | | |

TABLE II . REACTIVITY OF MOUSE MONOCLONAL ANTIBODIES TO BLOOD

GROUP ANTIGENS. SEROLOGICAL TEST WITH CULTURED HUMAN CELLS [a]

| CELLS | T-174 (Le[a]) | T-218 (Le[b]) | P-12 (X) | F-3 (Y) | H-11 (H-2) |
|---|---|---|---|---|---|
| Immunoglobulin subclass | $\gamma$-1 | $\mu$ | $\mu$ | $\mu$ | $\mu$ |
| **CANCER CELL LINES** | | | | | |
| **COLON** | | | | | |
| HT-29,SW-480,SW-403 | ● ● ● | ● ● ● | ◒ ● ◒ | ● ● ● | ○ ◒ ○ |
| SW-48,CACO-2,SW-1116 | ○ ◒ ● | ◒ ◒ ● | ● ● ◒ | ● ● ◒ | ○ ○ ○ |
| SK-CO-10,SK-CO-13 | ● ● | ● ◒ | ● ◒ | ● ● | ◒ ○ |
| SW-1417,SW-1222,SK-CO-15 | ● ◒ ○ | ◒ ◒ ○ | ◒ ◒ ◒ | ○ ◒ ● | ○ ○ ● |
| SW-620,SW-837,SK-CO-11 | ◒ ○ ○ | ● ○ ○ | ● ○ ◒ | ○ ○ ○ | ◒ ◒ ○ |
| SW-1083,SK-CO-12,SK-CO-1 | ◒ ○ ○ | ○ ○ ○ | ◒ ● ◒ | ◒ ● ◒ | ○ ○ ○ |
| **PANCREAS** | | | | | |
| ASPC-1,CAPAN-1,CAPAN-2 | ○ ○ ● | ○ ○ ● | ○ ○ ◒ | ○ ● ● | ○ ○ ○ |
| **HEPATIC AND BILIARY** | | | | | |
| SK-HEP-1,SK-CHL-1 | ○ ○ | ○ ○ | ○ ○ | ○ ○ | ○ ○ |
| **LUNG** | | | | | |
| SK-LC-1,-2,-4 | ○ ○ ○ | ○ ◒ ○ | ○ ● ◒ | ○ ● ● | ◒ ◒ ○ |
| SK-LC-5,-6,-8 | ○ ○ ○ | ○ ○ ○ | ● ● ◒ | ○ ● ● | ◒ ○ ○ |
| SK-LC-9,-10,-12 | ● ○ ◒ | ◒ ○ ◒ | ◒ ○ ● | ● ◒ ◒ | ○ ○ ● |
| SK-LC-15,-16,-17 | ● ○ ○ | ◒ ◒ ○ | ● ◒ ◒ | ◒ ◒ ● | ○ ◒ ○ |
| SK-LC-19,-23,-24 | ○ ○ ○ | ◒ ○ ○ | ○ ○ ○ | ● ● ● | ● ○ ● |
| SK-LC-25,-28,-LL | ○ ○ ◒ | ● ◒ ○ | ○ ○ ◒ | ● ● ● | ● ● ○ |
| CALU-1,CALU-5,CALU-6 | ◒ ◒ ◒ | ○ ● ● | ◒ ◒ ● | ○ ● ● | ○ ○ ○ |
| SK-MES-1,SK-LU-1 | ○ ◒ | ○ ● | ○ ◒ | ○ ◒ | ○ ○ |

**BLADDER**

253-J,SW-780,TCCSUP    O O O   O O O   ● ● O   O ● O   O O O

5637,VM-CUB-1,VM-CUB-2   O O O   ● ● O   O ● O   ● ● ●   O ● O

VM-CUB-3,575-A,RT-4   O O O   O O O   O ● ●   ● ● O   O ● O

639-V,J-82   O O   O O   O ●   O O   O O

**BREAST**

MDA-MB-361,MCF-7,CaMa   O O ●   O ● ●   O ● ●   ● ● ●   ● ● O

SK-BR-3,MDA-MB-157,A1Ab   O O O   O O O   ● ● ●   ● O O   ● O O

MDA-MB-231,BT-20,SK-BR-7   O O O   O O O   O O O   O ● ●   O ● O

SK-BR-5,BT-00474,ZR-75-1   O O O   O O ●   ● ● O   ● ● ●   O ● O

**MELANOMA**

VM-88,MeWo,SK-MEL-13   O O O   O O O   O O O   O O O   O O O

SK-MEL-23,-27,-28   O O O   O O O   O O O   O O O   O O O

SK-MEL-29,-33,-37   O O O   O O O   O O O   O O O   O O O

SK-MEL-42,-64,-73   O O O   O O O   O O O   O O O   O O O

SK-MEL-90,-129,-133   O O O   O O O   O O O   O O O   O O O

SK-MEL-176   O   O   O   O   O

**ASTROCYTOMA**

SK-MG-1,-2,-3   O O O   O O O   O O O   O O O   O O O

SK-MG-4,-9,10   O O O   O O O   O O O   O O O   O O O

SK-MG-12,-13,-14   O O O   O O O   O O O   O O O   O O O

SK-MG-16,MS,U-343   O O O   O O O   O O O   O O O   O O O

A-582   O   O   O   O   O

**NEUROBLASTOMA**

MC-NB-1,SMS-KAN,SK-N-MC   O O O   O O O   O ● ●   O O O   O O O

SMS-SAN,SKN-BE(2)   O O   O O   ● O   O O   O O

0248147

| RENAL CANCER | | | | | | | | | | | | | | |
|---|---|---|---|---|---|---|---|---|---|---|---|---|---|---|
| SK-RC-1,-2,-4 | o | o | o | o | o | o | ● | ● | ● | o | o | o | o | o | o |
| SK-RC-7,-9,-10 | o | o | o | o | o | o | o | o | o | o | o | o | o | o | o |
| SK-RC-17,-18,-21 | o | o | o | o | o | o | ● | o | o | ● | o | o | ⊖ | o | o |
| SK-RC-26A,-26B,28 | o | o | o | o | o | o | o | o | ● | o | o | o | o | o | o |
| SK-RC-29,-35,-37 | o | o | o | o | o | o | ⊖ | o | o | o | o | o | o | o | o |
| SK-RC-39,-42,-44 | o | o | o | o | o | o | ⊖ | o | ● | o | o | o | o | o | o |
| SK-RC-45,-48 | o | o | | o | o | | ⊖ | o | | o | o | | o | o | |

**B CELL LEUKEMIA**

| | | | | | | | | | | | | | | | |
|---|---|---|---|---|---|---|---|---|---|---|---|---|---|---|---|
| ARH 77 AG,ARA 10,DAUDI | o | o | o | o | o | o | o | o | o | o | o | o | o | o | o |
| SK-LY-16,-18,BALL-1 | o | o | o | o | o | o | o | o | o | o | o | o | o | o | o |
| SK-DHL-2,SKO-OO7,RAJI | o | o | o | o | o | o | ⊖ | o | o | ⊖ | o | o | ⊖ | o | o |
| LICR LON,UC 729-6 | o | o | o | o | o | o | o | o | ● | o | o | o | o | o | o |

**T CELL LEUKEMIA**

| | | | | | | | | | | | | | | | |
|---|---|---|---|---|---|---|---|---|---|---|---|---|---|---|---|
| HPB ALL,T-45,MOLT-4 | o | o | o | o | o | o | ● | ● | ● | o | o | o | o | o | o |
| CCRF-HSB 2,CCRF-CEM,P-12 | o | o | o | o | o | o | ⊖ | ● | ● | ● | o | ● | ● | o | ⊖ |

**MONOCYTE CELL LEUKEMIA**

| | | | | | | | | | | | | | | | |
|---|---|---|---|---|---|---|---|---|---|---|---|---|---|---|---|
| HL60,K-562,KG-1-G | o | o | o | o | o | o | ● | o | o | o | o | o | o | o | o |

**NULL CELL LEUKEMIA**

| | | | | | | | | | | | | | | | |
|---|---|---|---|---|---|---|---|---|---|---|---|---|---|---|---|
| NALM-1,NALM-16,NKL 1 | o | o | o | o | o | o | o | o | o | o | ⊖ | o | o | ⊖ | o |
| NKL 2,NALL 1 | o | o | | ⊖ | o | | ● | ● | | ⊖ | ● | | ⊖ | ● | |

**OVARIAN CANCER**

| | | | | | | | | | | | | | | | |
|---|---|---|---|---|---|---|---|---|---|---|---|---|---|---|---|
| SK-OV-6,SK-OV-4,A-7 | o | o | o | o | ● | o | o | ● | o | ● | ● | o | o | o | o |
| SW-626 | ● | | | ● | | | ● | | | ⊖ | | | ● | | |

**TERATOCARCINOMA**

| | | | | | | | | | | | | | | | |
|---|---|---|---|---|---|---|---|---|---|---|---|---|---|---|---|
| 577MF,Tera-1,833 KE | o | o | o | o | o | o | o | o | o | o | o | o | o | o | o |

Table II (continued 4)

-42-

0248147

CHORIOCARCINOMA

GCC-SV(O),OCC-M

CULTURED NORMAL CELLS

NORMAL FIBROBLAST

#1,#2,#3       O O O    O O O    O O O    O O O    O O O

#4,#5,#6       O O O    O O O    O O O    O O O    O O O

#7,#8          O O      O O      O O      O O      O O

NORMAL KIDNEY CELLS

#1,#2,         O O      O O      ● ●      O O      O O

---

a The symbols listed under the antibodies refer to titer against the cell line in the corresponding position in the left hand side of the table. The titer of the antibody was defined as the highest dilution producing at least 50% rosetting in the MHA assay (IgG antibody) or in the CF assay (IgM antibody) ●, $1 \times 10^{-3}$ – $1 \times 10^{-6}$; O, positive reaction but with under 50% rosetting at $10^{-3}$ dilution of antibody; $\overline{O}$, positive only with the absorption test; O no reactivity at antibody dilution of $5 \times 10^{-2}$.

TABLE III. BLOOD GROUP ANTIGEN EXPRESSION IN COLON CANCER AND IN NORMAL COLONIC MUCOSA

| Tumor location and specimen | Pathology grading [b] | Blood type | Antigen [a] | | | |
|---|---|---|---|---|---|---|
| | | | $Le^a$ | $Le^b$ | X | Y |
| **RECTUM** | | | | | | |
| AR | 2 | B | + | ● | + | - |
| AY | 1 | O | + | ● | + | + |
| BT | 2 | O | + | ● | + | + |
| BU | 3 | A | - | - | - | - |
| CN | 1 | O | + | + | + | + |
| DH | 3 | B | + | ● | + | ● |
| FN | 2 | AB | + | ● | - | ● |
| GV | ND | O | O | + | + | ● |
| HR | 2 | A | + | + | + | ● |
| MK | 2 | O | + | + | O | + |
| PP | 3 | A | O | ● | + | ● |
| RT | 2 | O | + | + | + | ● |
| RV | 2 | O | + | + | + | + |
| VT | 2 | O | + | + | + | ● |
| **RECTOSIGMOID** | | | | | | |
| CN | 3 | O | O | + | + | ● |
| DW | 2 | A | + | - | + | + |
| FG | 2 | A | + | ● | + | ● |
| GF | 2 | A | + | ● | + | + |

Table III (continued 2)

-44-

0248147

| | | | | | | |
|---|---|---|---|---|---|---|
| NC | 2 | A | − | − | − | − |
| NM | Mucinous | A | + | + | ● | ● |
| OA | 2 | A | + | O | + | ● |
| RB | 2 | A | + | ● | + | ● |
| **SIGMOID** | | | | | | |
| GC | 2 | A | + | ● | + | + |
| HT | 2 | O | + | + | + | + |
| MS | 2 | AB | + | ● | + | + |
| PG | 2 | A | + | − | + | ● |
| RM | 3 | O | − | − | ● | ● |
| **LEFT COLON** | | | | | | |
| BW | 1 | O | + | ● | + | + |
| EL | 2 | A | + | + | + | + |
| HO | 1 | B | + | + | + | ● |
| MY | 2 | O | + | + | + | ● |
| SI | 2 | A | − | − | + | + |
| SB | ND | A | + | ● | + | + |
| TN | 2 | B | + | − | + | ● |
| **TRANSVERSE COLON** | | | | | | |
| HM | 2 | B | + | + | + | ● |
| **RIGHT COLON** | | | | | | |
| BW | 2 | B | + | ● | + | ● |
| BG | 3 | O | O | + | + | + |
| FU | 2 | A | + | ● | + | ● |
| HT | 2 | AB | + | + | ● | + |

| | | | | | | | |
|---|---|---|---|---|---|---|---|
| AP | 2 | O | O | − | + | + | + 0248147 |
| FR | 2 | B | + | + | + | ● | |
| KP | 2 | O | + | + | + | ● | |

-------------------------------------------------------------

a
The symbols listed under the blood group substances refer to the reactivity of carcinoma and normal tissue sections by immuno-peroxidase staining:

−: not reactive either with tumor or with normal adjacent colon.

+: reactive with both tumor and normal adjacent colon.

O: reactive with normal adjacent colonic mucosa but not reactive with tumor tissue sections.

●: reactive with tumor but not reactive with normal adjacent colonic mucosa.

b

Pathology grading: 1, well differentiated; 2, moderately well differentiated; 3, poorly differentiated.

**TABLE IV.** EXPRESSION OF BLOOD GROUP ANTIGENS IN SALIVA, NORMAL COLON AND TUMORS OF COLORECTAL CANCER PATIENTS [a]

| No. | Patient Blood Group | Le^a Histology [c] S[b] | Le^a Histology N | Le^a Histology T | Le^a Glycolipid [d] N | Le^a Glycolipid T | Le^b Histology S | Le^b Histology N | Le^b Histology T | Le^b Glycolipid N | Le^b Glycolipid T | X Histology S | X Histology N | X Histology T | X Glycolipid N | X Glycolipid T | Y Histology S | Y Histology N | Y Histology T | Y Glycolipid N | Y Glycolipid T |
|---|---|---|---|---|---|---|---|---|---|---|---|---|---|---|---|---|---|---|---|---|---|
| 1[e] | B | ● | ● | ● | ● | ● | ○ | ○ | ○ | ○ | ○ | ● | ● | ● | ○ | ● | ○ | ○ | ● | ○ | ● |
| 2 | A | ○ | ● | ○ | ● | ○ | ● | ○ | ● | ○ | ● | ○ | ● | ○ | ● | ○ | ● | ○ | ● | ○ | ● |
| 3 | A | ○ | ● | ● | ● | ● | ● | ○ | ● | ○ | ● | ○ | ● | ○ | ○ | ● | ● | ○ | ● | ○ | ● |
| 4 | B | ● | ● | ● | ● | ● | ○ | ○ | ● | ○ | ● | ● | ● | ● | ○ | ● | ○ | ○ | ● | ○ | ● |
| 5 | A | ○ | ● | ○ | ● | ○ | ● | ○ | ○ | ○ | ● | ○ | ○ | ● | ○ | ● | ● | ○ | ○ | ○ | ● |
| 6 | O | ○ | ○ | ○ | | | ○ | ○ | ○ | | | ○ | ● | ● | | | ● | ○ | ● | | |
| 7 | B | ○ | ● | ○ | | | ○ | ○ | ○ | | | ○ | ● | ● | | | ● | ○ | ● | | |

Table IV Continued

a Not included in Table III

b Expression of blood groups in saliva glycoproteins as determined by ELISA. A positve reaction (●) for Le$^a$ indicates a "non-secretor" individual.

c Determined by immunoperoxidase assay on frozen and paraffin-fixed embedded sections of normal (N) and tumor (T) samples. ●: strong reactivity; ◒: weak reactivity; O: no reactivity.

d Determined by immunostaining of glycolipids extracted from normal (N) and tumor (T) samples and reactive by thin layer chromatography. ●: strong reactivity; ◒: weak reactivity; O: no reactivity ( see also Figure 3).

e Tumors from patients 1, 2, 5, and 7 were located in the distal colon and those from patients 3, 4, and 6 were from the proximal colon.

## TABLE V

### DERIVATION AND SPECIFICITY OF MOUS MONOCLONAL ANTIBODIES
### IDENTIFYING BLOOD GROUP ANTIGENS

| ANTIBODY (Ig subclass) | IMMUNIZING CELL LINE | BLOOD GROUP SPECIFICITY |
|---|---|---|
| T36 ($\gamma$3) | HT29 Colon Cancer | A (Type 1 and 2 Chains) |
| S8 ($\mu$) | SK-RC-7 Renal Cancer | B (Type 2 Chain) |
| K21 ($\mu$) | Tera-1 Teratocarcinoma | Precursor (Type 1 Chain) |
| T174 ($\gamma$1) | SK-CO-10 Colon Cancer | Lewis a ($Le^a$ – Type 1 Chain) |
| T218 ($\mu$) | SK-CO-10 Colon Cancer | Lewis b ($Le^b$ – Type 1 Chain) |
| P12 ($\mu$) | Fresh Human Placenta | Lewis x (X – Type 2 Chain) |
| F3 ($\mu$) | SK-LU-3 Lung Cancer | Lewis y (Y – Type 2 Chain) |

Note: Purified agglutinin I from <u>Ulex</u> <u>europeus</u> (Vector Laboratories) served to identify the H-antigen.

# TABLE VI

ANTIBODIES AND LECITIN (UEA) DETECTING BLOOD GROUP ANTIGENS

| NORMAL TISSUES | PRECURSOR | | A | | B | | H | | Le[a] | | Le[b] | | X | | Y | |
|---|---|---|---|---|---|---|---|---|---|---|---|---|---|---|---|---|
| | K21 | | H29-36 | | S8 | | UEA | | T-174 | | T-218 | | P12 | | F3 | |
| | F | A[1] | F | A | F | A | F | A | F | A | F | A | F | A | F | A |
| ESOPHAGUS | | | | | | | | | | | | | | | | |
| Basal cells | 0 | 0[2] | 0 | 0 | 0 | 0 | 0 | 0 | 0 | 0 | 0 | 0 | ● | 0 | ● | ● |
| Suprabasal cells | ● | 0 | 0 | 0 | 0 | 0 | 0 | 0 | ● | ● | ● | ● | ● | ● | ● | ● |
| STOMACH | | | | | | | | | | | | | | | | |
| Deep pit cells | ● | ● | 0 | 0 | 0 | 0 | 0 | 0 | ● | ● | ● | ● | ● | ● | ● | ● |
| Chief cells | ● | ● | 0 | 0 | 0 | 0 | 0 | 0 | ● | 0 | ● | ● | ● | ● | ● | ● |
| Parietal cells | ● | ● | 0 | 0 | 0 | 0 | 0 | 0 | ● | 0 | ● | ● | ● | ● | ● | ● |
| SMALL INTESTINE | | | | | | | | | | | | | | | | |
| Crypt cells | 0 | 0 | ● | 0 | ● | 0 | ● | ● | 0 | 0 | 0 | 0 | ● | ● | ● | ● |
| Paneth's cells | 0 | 0 | 0 | 0 | 0 | 0 | 0 | 0 | 0 | 0 | 0 | 0 | 0 | 0 | ● | ● |
| Goblet cells | ● | ● | 0 | 0 | 0 | 0 | 0 | 0 | ● | ● | ● | ● | 0 | 0 | 0 | 0 |
| Absorptive cells | ● | ● | 0 | 0 | 0 | 0 | 0 | 0 | ● | ● | ● | ● | 0 | 0 | 0 | 0 |
| COLON | | | | | | | | | | | | | | | | |
| Crypt cells | 0 | 0 | ● | 0 | ● | 0 | ● | ● | ● | ● | 0 | 0 | ● | ● | 0 | 0 |
| Goblet cells | 0 | 0 | 0 | 0 | 0 | 0 | 0 | 0 | ● | ● | 0 | 0 | 0 | 0 | 0 | 0 |
| Absorptive cells | ● | ● | 0 | 0 | 0 | 0 | 0 | 0 | ● | ● | 0 | 0 | ● | ● | 0 | 0 |

0248147

1) F: Fetal Tissues; A: Adult Tissues.

2) ●: Homogeneously stained; ◑: Heterogeneously stained; 0: Negative.

3) Analysis of formalin-fixed paraffin-embedded tissue sections using immunoperoxidase (avidin-biotin) techniques.

TABLE VII·

-50 0248147

| PATIENT | AGE | SEX | BLOOD TYPE | PRIMARY TUMOR | METASTATIC TUMOR | DUKES' STAGE |
|---------|-----|-----|------------|---------------|------------------|--------------|
| Case 1 | 49 | F | A | Sigmoid | Lymph node | C |
| Case 2 | 61 | F | A | Rectum | Lymph node | C |
| Case 3 | 61 | M | AB | Sigmoid | Lymph node | C |
| Case 4 | 68 | M | B | Sigmoid | Lymph node | C |
| Case 5 | 73 | M | A | Left colon | Lymph node | C |
| Case 6 | 73 | M | O | Cecum | Lymph node | C |
| Case 7 | 55 | F | O | Rectum | Lymph node | C |
| Case 8 | 64 | M | A | Rectum | Lymph node | C |
| Case 9 | 45 | F | A | Rectum | Lymph node | C |
| Case 10 | 59 | M | O | Rectum | Lymph node | C |
| Case 11 | 49 | F | A | Right colon | Lymph node | C |
| Case 12 | 28 | F | O | Rectum | Lymph node | C |
| Case 13 | 61 | M | B | Cecum | Lymph node | C |
| Case 14 | 56 | M | A | Sigmoid | Liver | B |
| Case 15 | 63 | F | B | Sigmoid | None | B |
| Case 16 | 64 | F | O | Transverse colon | None | B |
| Case 17 | 76 | F | O | Cecum | None | B |
| Case 18 | 72 | F | B | Left colon | None | B |
| Case 19 | 68 | F | O | Transverse colon | None | B |
| Case 20 | 52 | M | O | Rectum | None | A |

# ANTIBODIES AND LECTINS DETECTING BLOOD GROUP ANTIGENS

| | PRECURSOR K21 | A H29-36 | B S8 | N UEA | Le$^a$ T-174 | Le$^b$ T-218 | X P12 | Y F3 |
|---|---|---|---|---|---|---|---|---|
| TISSUES: | NPM [1] | NPM | NPM | NPM | NPM | NPM | NPM | NPM |
| Case 1 | ●●● [2] | ●●● | 000 | ●●● | ●●● | 0●● | ●●● | 0●● |
| Case 2 | ●●● | 000 | 000 | 0●● | ●●● | 0●● | ●●● | 0●● |
| Case 3 | 0●● | 0●0 | 0●0 | 0●● | ●●● | 0●● | 0●● | 0●● |
| Case 4 | ,0●● | 000 | 000 | ●●● | ●●● | 0●● | 0●● | 0●● |
| Case 5 | ●●● | 0●● | 000 | 0●● | 0●0 | 000 | ●●● | 0●● |
| Case 6 | 0●● | 000 | 000 | ●●0 | ●●● | 0●● | 0●● | 0●● |
| Case 7 | ●●● | 000 | 000 | 0●● | ●●● | 000 | ●●● | 0●● |
| Case 8 | ●●● | 0●● | 000 | ●●● | ●●● | 0●● | 0●● | 0●● |
| Case 9 | ●●● | 0●● | 000 | 0●● | ●●● | 0●● | ●●● | 0●● |
| Case 10 | ●●● | 000 | 000 | 0●● | ●00 | ●●● | ●●● | 000 |
| Case 11 | ●●● | ●00 | 000 | ●●● | ●●● | ●●● | ●●● | 0●● |
| Case 12 | 0●● | 000 | 000 | ●●● | ●●0 | ●●● | ●●● | 0●● |
| Case 13 | ●00 | 000 | ●●0 | ●●● | ●●● | 0●● | ●●● | 0●● |
| Case 14 | 00● | 000 | 000 | 00● | ●0● | 00● | 0●● | 0●● |
| | | | | | | | | |
| Case 15 | 00 | 00 | 00 | ●● | ●● | 0● | ●● | 0● |
| Case 16 | 0● | 00 | 00 | ●● | ●● | 0● | ●● | 0● |
| Case 17 | ●● | 00 | 00 | 0● | ●0 | 0● | ●● | 0● |
| Case 18 | 0● | 00 | 00 | 0● | ●● | 0● | ●● | 0● |
| Case 19 | 0● | 00 | 00 | 0● | ●● | 0● | ●● | 0● |
| Case 20 | 0● | 00 | 00 | ●● | ●● | 0● | ●● | 0● |

[1] N: Normal colonic mucosa; P: Primary colon tumor; M: Metastatic colonic tumor.

[2] ●: Homogeneously stained; ◑: Heterogeneously stained; 0: Negative.

[3] Analysis of formalin-fixed paraffin-embedded tissue sections using immunoperoxidase (avidin-biotin) techniques.

## TABLE IX

IMMUNOPEROXIDASE STAINING OF HUMAN TISSUES AND SECRETIONS WITH
MONOCLONAL ANTIBODIES DETECTING LEWIS BLOOD GROUP ANTIGENS IN
PATIENTS WITH KNOWN SECRETORY STATUS

| CASE # | BLOOD TYPE | SECRETOR STATUS | $Le^a$ RENTS[1] | $Le^b$ RENTS | X RENTS | Y RENTS |
|---|---|---|---|---|---|---|
| Case 1 | A | S | 00●●●[2] | 0000● | 000●● | 0000● |
| Case 2 | A | S | 00●●● | 0000● | 000●● | 0000● |
| Case 16 | B | S | 00●●● | 0000● | 000●● | 0000● |
| Case 17 | O | S | 00●00 | 0000● | 000●● | ●●000 |
| Case 4 | B | NS | 00●●● | 0000● | 00000 | 00000 |
| Case 15 | B | NS | 00●●● | 00000 | 000●● | 00000 |

1) R: Erythrocytes; E: Endothelial cells; N: Normal colonic
   mucosa; T: Tumor cells; S: Luminal secretions.

2) ●: Homogeneously stained; 0: Heterogeneously stained; O: Negative.

## CLAIMS

1.  A method of determining the presence of cancer cells in a sample comprising contacting said sample with at least one monoclonal antibody specific for a blood group antigen under conditions favoring formation of complexes between said antibody and the blood group antigen for which it is specific when said antigen is expressed by said cancer cells, and determining the presence of said complexes.

2.  A method as in Claim 1, wherein said sample is contacted with a panel of at least two monoclonal antibodies.

3.  A method as in Claim 2, wherein said monoclonal antibodies are selected from the group consisting of T-174, T-15, T-85, T-307, H-68, T-218, P-12, F-3, H-11, K-21, H-29-36, S8, and UEA.

4.  A method as in Claim 1, wherein said sample comprises colon cancer cells.

5.  A method as in Claim 4, wherein said colon cancer cell containing sample is contacted with at least one monoclonal antibody specific for the X antigen.

6.  A method as in Claim 1, wherein said sample comprises breast cancer cells.

7. A method as in Claim 6, wherein said breast cancer cell containing sample is contacted with at least one monoclonal specific for the X antigen.

8. A method as in Claim 1, wherein said sample comprises lung cancer cells.

9. A method as in Claim 8, wherein said lung cancer cell containing sample is contacted with at least one monoclonal antibody of specific for the X antigen.

10. A method as in Claim 1, wherein said sample comprises ovarian cancer cells.

11. A method as in Claim 10, wherein said ovarian cancer cell containing sample is contacted with at least one monoclonal antibody specific for the X antigen.

12. A method as in Claim 1, wherein said sample comprises choriocarcinoma cells.

13. A method as in Claim 12, wherein said choriocarcinoma cell containing sample is contacted with at least one monoclonal antibody specific for X antigen.

14. A method as in Claim 1, wherein said sample comprises monocyte or null cell derived leukemia cells.

15.    A method as in Claim 14, wherein said leukemia cell containing sample is contacted with at least one monoclonal antibody specific for the X antigen.

16.    A method as in Claim 4, wherein said colon cancer containing sample is contacted with at least one monoclonal antibody specific for the Y antigen.

17.    A method as in Claim 6, wherein said breast cancer containing sample is contacted with at least one monoclonal antibody specific for the Y antigen.

18.    A method as in Claim 8, wherein said lung cancer containing sample is contacted with at least one monoclonal antibody specific for the Y antigen.

19.    A method as in Claim 10, wherein said ovarian cancer containing sample is contacted with at least one monoclonal antibody specific for the Y antigen.

20.    A method as in Claim 6, wherein said breast cancer containing sample is contacted with at least one monoclonal antibody specific for H-2 antigen.

21.    A method as in Claim 8, wherein said lung cancer containing sample is contacted with at least one monoclonal antibody specific for H-2 antigen.

22. A method as in Claim 4, wherein said colon cancer cell sample is contacted with at least one monoclonal antibody specific for Lewis A antigen.

23. A method as in Claim 4, wherein said colon cancer cell sample is contacted with at least one monoclonal antibody specific for Lewis B antigen.

24. A method as in Claim 8, wherein said lung cancer cell sample is contacted with at least one monoclonal antibody specific for Lewis B antigen.

25. A method as in Claim 1, wherein said sample comprises bladder cancer cells.

26. A method as in Claim 25, wherein said bladder cell sample is contacted with at least one monoclonal antibody specific for X antigen.

27. A method as in Claim 1, wherein said sample comprises gastrointestical cancer cells.

28. A method as in Claim 27, wherein said gastrointestinal cancer cells comprise cancer cells selected from the group consisting of esophagus, stomach, and small intestine cancer cells.

29. A method as in Claim 27, wherein said sample is contacted with at least one monoclonal antibody specific for precursor type I antigen.

30. A method as in Claim 27, wherein said sample is contacted with at least one monoclonal antibody specific for type H antigen.

31. A method as in Claim 27, wherein said sample is contacted with at least one monoclonal antibody specific for Lewis A antigen.

32. A method as in Claim 27, wherein said sample is contacted with at least one monoclonal antibody specific for Lewis B antigen.

33. A method as in Claim 27, wherein said sample is contacted with at least one monoclonal antibody specific for type X antigen.

34. A method as in Claim 27, wherein said sample is contacted with at least one monoclonal antibody specific for type Y antigen.

35. A method as in Claims 1, 2, 4, 5, 6, 7, 8, 9, 10, 11, 12, 13, 14, 15, 25, 26, 27, 28 or 33, wherein said cell sample is contacted with monoclonal antibody P-12.

36. A method as in Claims 1, 2, 4, 6, 8, 10, 16, 17, 18, 19, 27, 28, or 34, wherein said cell sample is contacted with monoclonal antibody F-3.

37. A method as in Claims 1, 2, 6, 8, 20, 21, 27, 28, or 30, wherein said cell sample is contacted with monoclonal antibody H-11.

38. A method as in Claims 1, 2, 4, 22, 27, 28, or 31, wherein said cell sample is contacted with monoclonal antibody T-174.

39. A method as in Claims 1, 2, 4, 8, 23, 24, 27, 28 or 32, wherein said cell sample is contacted with monoclonal antibody T-218.

40. A panel of monoclonal antibodies useful in determining the presence of blood group antigens on carrier cells comprising at least one monoclonal antibody from the group consisting of K21, H29-36, S8, T-174, T-218, P-12, F-3, T-218, and H-11.

41. A panel as in Claim 40, wherein said panel determines the presence of precursor type I antigen and comprises monoclonal antibody K21.

42. A panel as in Claim 40, wherein said panel determines the presence of type A antigen and comprises monoclonal antibody H29-36.

43. A panel as in Claim 40, wherein said panel determines the presence of type B antigen and comprises monoclonal antibody S8.

44. A panel as in Claim 40, wherein said panel determines the presence of Lewis A type antigen and comprises monoclonal antibody T-174.

45. A panel as in Claim 40, wherein said panel determines the presence of Lewis B type antigen and comprises monoclonal antibody T-218.

46. A panel as in Claim 40, wherein said panel determines the presence of type X antigen and comprises monoclonal antibody P-12.

47. A panel as in Claim 40, wherein said panel determines the presence of type Y antigen and comprises monoclonal antibody F3.

48.  A panel as in Claim 40, wherein said panel determines the presence of type H-2 antigen and comprises monoclonal antibody H-11.

## TYPE 1 CHAINS

Galβ(1→3)GlcNAc -
  2
  ↑
Fucα1         H-1

Galβ(1→3)GlcNAc -
  4
  ↑
Fucα1        Le [a]

Galβ(1→3)GlcNAc -
  2      4
  ↑      ↑     b
Fucα1   Fucα1   Le

## TYPE 2 CHAINS

Galβ(1→4)GlcNAc -
  2
  ↑
Fucα1        H-2

Galβ(1→4)GlcNAc -
  3
  ↑
Fucα1        X

Galβ(1→4)GlcNAc -
  2      3
  ↑      ↑
Fucα1   Fucα1   Y

FIGURE 1

FIGURE 2

3/21

0248147

FIGURE 3

4/21

0248747

FIGURE 4

# FIGURE 5

FIGURE 6 a

6121

0248147

FIGURE 6b

FIGURE 6C

FIGURE 6 d

0248147

FIGURE 7a

FIGURE 7b

0248147

FIGURE 7c

0248147

FIGURE 7 d

14/21    0248147

FIGURE 7e

FIGURE 7f

16/21    0248147

FIGURE 7g

0248147

FIGURE 7h

18/21

0248147

FIGURE 8a

19/21

0248147

FIGURE 8b

0248147

FIGURE 8c

FIGURE 8d